# EUROPEAN PATENT SPECIFICATION

(11) **EP 1 173 432 B1**
(45) Date of publication and mention of the grant of the patent: **17.10.2007**
(21) Application number: 99915418.0
(22) Date of filing: 21.04.1999
(51) Int. Cl.: C07D 401/04

(54) **PIPERIDINE-INDOLE COMPOUNDS HAVING 5-HT6 AFFINITY**
PIPERIDIN-INDOL DERIVATE MIT 5-HT6 AFFINITÄT
COMPOSES PIPERIDINE-INDOL AYANT UNE AFFINITE AVEC 5-HT6

(43) Date of publication of application: 23.01.2002
(73) Proprietor: NPS Allelix Corp., Mississauga Ontario, L4V 1V7 (CA)
(72) Inventor: SLASSI, Abdelmalik, Mississauga, Ontario L5N 5G4 (CA); EDWARDS, Louise, Mississauga, Ontario L5H 2C6 (CA); O'BRIEN, Anne, Upper Toronto, Ontario M6P 1E2 (CA); XIN, Tao, Woodbridge, Ontario L4H 2B1 (CA); TEHIM, Ashok, Mississauga, Ontario L5N 7H1 (CA)
(74) Representative: Smaggasgale, Gillian Helen
(86) International application number: PCT/CA1999/000342
(87) International publication number: WO 2000/063203

(56) References cited:
- EP-A- 0 200 322
- EP-A- 0 465 398
- WO-A-92/13856
- WO-A-97/47302
- MONSMA ET AL: "Cloning and Expression of A Novel Serotonin Receptor with High Affinity for Tricyclic Psychotropic Drugs" MOLECULAR PHARMACOLOGY,US,BALTIMORE, MD, vol. 43, no. 3, page 320-327 XP002093842 ISSN: 0026-895X
- SAUDOU F ET AL: "5-HT RECEPTOR SUBTYPES: MOLECULAR AND FUNCTIONAL DIVERSITY" MEDICINAL CHEMISTRY RESEARCH,US,BIRKHAEUSER, BOSTON, vol. 4, no. 1, page 16-84 XP000604196 ISSN: 1054-2523
- SLEIGHT ET AL: "The 5-hydroxytryptamine-6 receptor: localisation and function" EXPERT OPINION ON THERAPEUTIC PATENTS,GB,ASHLEY PUBLICATIONS, vol. 8, no. 10, page 1217-1224 XP002093843 ISSN: 1354-3776
- MARTIN G R ET AL: "The structure and signalling properties of 5-HT receptors: an endless diversity?" TRENDS IN PHARMACOLOGICAL SCIENCES,GB,ELSEVIER TRENDS JOURNAL, CAMBRIDGE, vol. 19, no. 1, page 2-4 XP004107578 ISSN: 0165-6147
- HOYER D AND MARTIN G: "5-HT receptor classification and nomenclature: towards a harmonization with the human genome" NEUROPHARMACOLOGY,GB,PERGAMON PRESS, OXFORD, no. 36, page 419-428 XP002075372 ISSN: 0028-3908
- HOYER D ET AL: "VII. INTERNATIONAL UNION OF PHAMACOLOGY CLASSIFICATION OF RECEPTORS FOR 5-HYDROXYTRYPTAMINE (SEROTONIN)" PHARMACOLOGICAL REVIEWS,US,WILLIAMS AND WILKINS INC., BALTIMORE, MD,, vol. 46, no. 2, page 157-203 XP000604197 ISSN: 0031-6997

## Description

This invention relates to indole compounds having affinity for the serotonin 5-HT₆ receptor, to pharmaceutical compositions containing them and to their medical use, particularly in the treatment of CNS conditions.

According to one aspect of the invention, there are provided compounds of Formula I and salts, solvates or hydrates thereof: 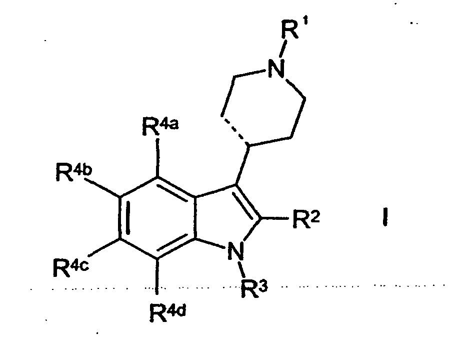 wherein:
R¹ is selected from the group consisting of H and C₁₋₄alkyl;
R² is selected from the group consisting of H, C₁₋₄alkyl and benzyl; represents a single or double bond;
R³ is SO₂R⁵;
R^{4a} is selected from the group consisting of H, OH, halo, C₁₋₄alkyl and C₁₋₄alkoxy;
R^{4b} is selected from the group consisting of H, hydroxy, halo,
C₃₋₇cycloalkyloxy, C₁₋₄alkoxy, C₁₋₄alkyl, benzyloxy, phenoxy, trifluoromethyl, trifluoromethoxy and vinyl;
R^{4c} is selected from the group consisting of H, OH, halo, C₁₋₄alkyl and C₁₋₄alkoxy;
R^{4d} is selected from the group consisting of H, OH, halo, C₁₋₄alkyl and C₁₋₄alkoxy; and
R⁵ is selected from the group consisting of phenyl, pyridyl, thienyl, quinolinyl and naphthyl which are optionally substituted with 1-4 substituents selected from C₁₋₄alkoxy, C₁₋₄alkyl, halo, nitro, trifluoromethyl, trifluoromethoxy, 1-2-methylenedioxy, C₁₋₄alkylcarbonyl, C₁₋₄alkoxycarbonyl and C₁₋₄alkylS-.

According to another aspect of the invention, there is provided a pharmaceutical composition comprising a compound of Formula I in an amount effective to antagonize the 5-HT₆ receptor, and a pharmaceutically acceptable carrier.

In another aspect of the present invention there are provided compositions containing the present compounds in amounts for pharmaceutical use to treat CNS conditions where a 5-HT₆ ligand is indicated, for example, for the treatment or prevention of central nervous system disturbances such as psychosis, schizophrenia, manic depression, depression, neurological, disturbances, memory disturbances, Parkinsonism, amylotrophic lateral sclerosis, Alzheimer's disease and Huntington's disease. These and other aspects of the present invention are described in greater detail hereinbelow.

Prior art which discloses indole derivatives having neurochemical inhibitory activity, or otherwise revelant to 5HT receptors, includes the following:
WO-A-97/47302
EP-A-0200322
EP-A-0465398
WO-A-92/13856
Mol. Pharmacol. 43, 320 (1993)
Med. Chem. Res. 4,16 (1964)
Exp. Opin. Ther. Patents 8,1217 (1998)
Trends Pharmacol. Sci. 19, 2 (1998)
Neuropharmacol. 36, 419 (1994)
Pharmacol. Rev. 46, 157 (1992)

The term halo as used herein means halogen and includes fluoro, chloro, bromo and the like.

The term "pharmaceutically acceptable salt" means an acid addition salt which is compatible with the treatment of patients.

A "pharmaceutically acceptable acid addition salt" is any non-toxic organic or inorganic acid addition salt of the base compounds represented by Formula I or any of its intermediates. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic, p-toluenesulfonic acid and other sulfonic acids such as methanesulfonic acid and 2-hydroxyethanesulfonic acid. Either the mono- or di-acid salts can be formed, and such salts can exist in either a hydrated, solvated or substantially anhydrous form. In general, the acid addition salts of these compounds are more soluble in water and various hydrophilic organic solvents, and generally demonstrate higher melting points in comparison to their free base forms. The selection criteria for the appropriate salt will be known to one skilled in the art.

"Solvate" means a compound of Formula I or the pharmaceutically acceptable salt of a compound of Formula I wherein molecules of a suitable solvent are incorporated in a crystal lattice. A suitable solvent is physiologically tolerable at the dosage administered as the solvate. Examples of suitable solvents are ethanol and the like.

The term "stereoisomers" is a general term for all isomers of the individual molecules that differ only in the orientation of their atoms in space. It includes mirror image isomers (enantiomers), geometric (cis/trans) isomers and isomers of compounds with more than one chiral centre that are not mirror images of one another (diastereomers).

The term "treat" or "treating" means to alleviate symptoms, eliminate the causation of the symptoms either on a temporary or permanent basis, or to prevent or slow the appearance of symptoms of the named disorder or condition.

The term "therapeutically effective amount" means an amount of the compound which is effective in treating the named disorder or condition.

The term "pharmaceutically acceptable carrier" means a non-toxic solvent, dispersant, excipient, adjuvant or other material which is mixed with the active ingredient in order to permit the formation of a pharmaceutical composition, i.e., a dosage form capable of administration to the patient. One example of such a carrier is a pharmaceutical acceptable oil typically used for parenteral administration.

The term "schizophrenia" means schizophrenia, schizophreniform disorder, schizoaffective disorder and psychotic disorder wherein the term "psychotic" refers to delusions, prominent hallucinations, disorganized speech or disorganized or catatonic behavior. See Diagnostic and Statistical Manual of Mental Disorder, fourth edition, American Psychiatric Association, Washington, D.C.

In embodiments of the invention, compounds of Formula I include those in which R¹ is selected from H and C₁₋₄alkyl. Preferably, R¹ is methyl. Also within the scope of the invention are compounds of Formula I wherein R² is selected from H, C₁₋₄alkyl and benzyl. In preferred embodiments, R² is H.

Compounds of the invention include Formula I compounds wherein R³ is SO₂R⁵. Within R³, R⁵ is selected from phenyl, pyridyl, thienyl, quinolinyl and naphthyl which are optionally substituted with 1-4 substituents selected from C₁₋₄alkoxy, C₁₋₄alkyl, halo, nitro, trifluoromethyl, trifluoromethoxy, 1,2-methylenedioxy, C₁₋₄alkylcarbonyl, C₁₋₄alkolcycarbonyl and C₁₋₄alkylS-. R⁵ is specifically selected from phenyl, naphthyl and thienyl which are all optionally substituted with 1-3 groups independently selected from methyl, methoxy, halo, trifluoromethyl and 1,2-methylenedioxy. In preferred embodiments, when R³ is SO₂R⁵, R⁵ is selected from the group consisting of naphthyl and phenyl optionally substituted with 1-3 substituents independently selected from chloro, bromo, fluoro, nitro, methyl and methoxy. In more preferred embodiments, when R³ is SO₂R⁵, R⁵ is selected from the group consisting of phenyl, 2-chlorophenyl, 2-bromophenyl, 1-naphthyl, 4-fluorophenyl, 4-chlorophenyl, 4-bromophenyl, 2,4-dichlorophenyl, 3-nitro-4-chlorophenyl, 2,4,6-trimethylphenyl, 4-methyoxyphenyl and 4-methylphenyl.

In further embodiments of the invention, R^{4b} is selected from H, hydroxy, halo, C₃₋₇cycloalkyloxy, C₁₋₄alkoxy, C₁₋₄alkyl, benzyloxy, phenoxy, trifluoromethyl, trifluoromethoxy and vinyl. In specific embodiments, R^{4b} is selected from H, halo, cyclohexyloxy trifluoromethoxy and trifluoromethyl. Preferably, R^{4b} is selected from chloro, trifluoromethoxy, trifluoromethyl and fluoro.

In other embodiments of the invention, R^{4a}, R^{4c} and R^{4d} are each independently selected from H, OH, halo, C₁₋₄alkyl and C₁₋₄alkoxy. In specific embodiments, R^{4a} and R^{4c} are both H and R^{4d} is halo or R^{4c} is halo and R^{4a}, R^{4b}, R^{4d} are all H. In a preferred embodiment, R^{4a}, R^{4c} and R^{4d} are all H.

In another embodiment of the invention, - represents a single or double bond. Preferably - represents a double bond.

In embodiments of the invention, the compounds of Formula I include:
5-Fluoro-1-(4-methylphenylsulfonyl)-3-(1-methyl-1,2,3,6-tetrahydro-4-pyridinyl)indole;
1-(4-Methoxyphenylsulfonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridinyl)indole;
1-(4-Bromophenylsulfonyl)-5-fluoro-3-(1-methyl-1,2,3,6-tetrahydro-4-pyridinyl)indole;
5-Fluoro-1-(2,5-dichlorophenylsulfonyl)-3-(1-methyl-1,2,3,6-tetrahydro-4-pyridinyl)indole;
1-(4-Chloro-3-nitrophenylsulfonyl)-5-fluoro-3-(1-methyl-1,2,3,6-tetrahydro-4-pyridinyl)indole;
5-Fluoro-3-(1-methyl-1,2,3,6-tetrahydro-4-pyridinyl)-1-(2,4,6-trimethylphenylsulfonyl)indole;
5-Fluoro-3-(1-methyl-1,2,3,tetrahydro-4-pyridinyl)-1-(2,4,6-triisopropylphenylsulfonyl)indole;
5-Cyclohexyloxy-1-(4-methylphenylsulfonyl)-3-(1-methyl-1,2,3,6 tetrahydro-4-pyridinyl)indole;
6-Bromo-1-(4-methoxyphenylsulfonyl)-3-(1-methyl-1,2,3,6-tetrahydro-4-pyridinyl)indole;
6-Bromo-1-(fluorophanylsulfonyl)-3-(1-methyl-1,2,3,6-tetrahydro-4-pyridinyl)indole;
5-Cyclohexyloxy-3-(1-methyl-1,2,3,6-tetrahydro-4-pyridinyl)-1-(1-naphthylsulfonyl)indole;
5-Cyclohexyloxy-3-(1-methyl-1,2,3,6-tetrahydro-4-pyridinyl)-1-(2 naphthylsulfonyl)indole:
1-(2-Bromophenylsulfonyl)-5-cyclohexyloxy-3-(1-methyl-1,2,3,4-tetrahydro-4-pyridinyl)indole;
5-Cyclohexyloxy-1-(2,5-dichlorophenylsulfonyl)-3-(1-methyl-1,2,3,6-tetrahydro-4-pyridinyl)indole;
5-Cyclohexyloxy-3-(1-methyl-1,2,3,6-tetrahydro-4-pyridinyl)-1-(2,4,6-trimethylphenylsulfonyl)indole;
5-Cyclohexyloxy-3-(1-methyl-1,2,3,6-tetrahydro-4-pyridinyl)-1-(2,4,6-triisopropylphenylsulfonyl)indole;
5-Cyclohexyloxy-3-(1-methyl-1,2,3,6-tetrahydro-4-pyridinyl)-1-(3-nitrophenylsulfonyl)indole;
1-(4-Chloro-3-nitrophenylsulfonyl)-5-cyclohexyloxy-3-(1-methyl-1,2,3,6-tetrahydro-4-pyridinyl)indole;
5-Cyclohexyloxy-3-(1-methyl-1,2,3,6-tetrahydro-4-pyridinyl)-1-(3-nitro-4-trifluoromethylphenylsulfonyl)indole:
1-(4-Bromophenylsulfonyl)-5-cyclohexyloxy-3-(1-methyl-1,2,3,6-tetrahydro-4-pyridinyl)indole;
5-Cyclohexyloxy-1-(4-methoxyphenylsulfonyl)-3-(1-methyl-1,2,3,6-tetrahydro-4-pyridinyl)indole;
5-Cyclohexyloxy-1-(4-fluorophenylsulfonyl)-3-(1-methyl-1,2,3,6-tetrahydro-4-pyridinyl)indole;
1-(4-Chlorophenylsulfonyl)-5-cyclohexyloxy-3-(1-methyl-1,2,3,6-tetrahydro-4-pyridinyl)indole;
1-(4 t-Butylphenylsulfonyl)-5-cyclohexyloxy-3-(1-methyl-1,2,3,6-tetrahydro-4-pyridinyl)indole;
1-(2-Bromophenylsulfonyl)-5-fluoro-3-(1-methyl-1,2,3,6-tetrahydro-4-pyridinyl)indole;
1-(4-Fluorophenylsulfonyl)-5-fluoro-3-1(methyl-1,2,3,6-tetrahydro-4-pyridinyl)indole;
1-(4-Chlorophenylsufonyl)-5-fluoro-3-(1-methyl-1,2,3,6-tetrahydro-4-pyridinyl)indole;
1-(4-^{t}Butylphenylsulfonyl)-4-fluoro-3-(1-methyl-1,2,3,6-tetrahydro-4-pyridinyl)indole;
5-Fluoro-3-(1-methyl-1,2,3,6-tetrahydro-4-pyridinyl)-1-(3-nitrophenylsulfonyl)indole;
5-Fluoro-3-(1-methyl-1,2,3,6-tehahydro-4-pyridinyl)-1-(1-naphthylsulfonyl)indole;
5-Fluoro-3-(1-methyl-1,2,3,6-tetrahydro-4-pyridinyl)-1-(2-naphthylsulfonyl)indole;
5-Chloro-1-(4-fluorophenylsulphonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridinyl)-5-trifluoromethoxyindole;
1-(4-t-Butylphenylsulfonyl)-3-(1-methyl-1,2,3,6-tetrahydro-4-pyridinyl)-5-trifluoromethoxyindole;
1-(4-Chlorophenylsulfonyl)-3-(1-methyl-1,2,3,6-tetrahydro-4-pyridinyl)-5-trifluoromethoxyindole;
1-(4-Methylphenylsulfonyl)-3-(1-methyl-1,2,3,6-tetrahydro-4-pyridinyl)-5-trifluoromethoxyindole;
(4-Methoxyphenylsulfonyl)-3-(1-methyl-1,2,3,6-tetrahydro-4-pyridinyl)-5-trifluoromethoxyindole;
1-(4-Fluorophenylsulfonyl)-3-(1-methyl-1,2,3,6-tetrahydro-4-pyridinyl)-5-trifluoromethylindole hydrochloride;
1-(4-t-Butylphenylsulfonyl)3-(1-methyl-1,2,3,6-tetrahydro-4-pyridinyl-5-trifluoromethylindole hydrochloride;
1-(4-Chlorophenylsulfonyl)-3-(1-methyl-1,2,3,6-tetrahydro-4-pyridinyl)-5-trifluaromethylindole hydrochloride;
1-(4-Methoxyphenylsulfonyl)-3-(1-methyl-1,2,3,6 tetrahydro-4-pyridinyl)-5-trifluoromethylindole hydrochloride;
1-(4-Methylphenylsulfonyl)-3-(1-methyl-1,2,3,6-tetrahydro-4-pyridinyl)-5-trifluoromethylindole hydrochloride;
5-Cyclohexyloxy-1-(4-methylphenysulfonyl)-3-(1-methyl-4-piperidinyl)indole;
5-Chloro-1-(4-fluorophenylsulfonyl)-3-(1-methyl-4-piperidinyl)indole;
1-(4-Fluorophenylsulfonyl)-3-(1-methyl-4-piperidinyl)indole;
1-(4-Fluorophenylsulfonyl)-6-chloro-3-(1-methyl-4-piperidinyl)indole;
1-(4-Fluorophenylsulfonyl)-5-fluoro-3-(1-methyl-4-piperidinyl)indole;
5,7-Difluoro-3-(1-methyl-1,2,3,6-tetrahydro-4-pyridinyl)-1-(4-methylphenylsulfonyl)indole;
5,7-Difluoro-1-(4-fluorophenylsulfonyl)-3-(1-methyl-1,2,3,6-tetrahydro-4-pyridinyl)indole;
1-(4-Bromophenylsulfonyl)-5,7-difluoro-3-(1-methyl-1,2,3,6-tetrahydro-4-pyridinyl)indole;
1-(4-Chlorophenylsulfonyl)-5,7-difluoro-3-(1-methyl-1,2,3,6-tetrahydro-4-pyridinyl)indole;
5,7-Difluoro-3-(1-methyl-1,2,3,6-tetrahydro-4-pyridinyl)-1-(1-naphthylsulfonyl)indole;
5,7-Difluoro-3-(1-methyl-1,2,3,6-tetrahydro-4-pyridinyl)-1-(2-naphthylsulfonyl)indole; and
5,7-Difluoro-3-(1-methyl-1,2,3,6-tetrahydro-4-pyridinyl)-1-(2,4,6-trimethylphenyl)sulfonylindole.

In specific embodiments of the invention, the compounds of Formula I include:
5-Fluoro-1-(4-methylphenylsulfonyl)-3-(1-methyl-1,2,3,6-tetrahydro-4-pyridinyl)indole;
1-(4-Methoxyphenylsulfonyl)-3-(1-methyl-1,2,3,8-tetrahydro-4-pyridinyl)indole;
1-(4-Fluorophenylsulfonyl)-3-(1-methyl-1,2,3,6-tetrahydro-4-pyridinyl)indole;
1-(4-Bromophenylsulfonyl)-5-fluoro-3-(1-methyl-1,2,3,6-tetrahydro-4-pyridinyl)indole;
5-Fluoro-1-(2,5-dichlorophenylsulfonyl)-3-(1-methyl-1,2,3,6-tetrahydro-4-pyridinyl)indole;
1-(4-Chloro-3-nitrophenylsulfonyl)-5-fluoro-3-(1-methyl-1,2,3,6-tetrahydro-4-pyridinyl)indole:
5-Fluoro-3-(1-methyl-1,2,3,6-tetrahydro-4-pyridinyl)-1-(2,4,6-trimethylphenylsulfonyl)indole:
5-Cyclohexyloxy-1-(4-methylphenylsulfonyl)-3-(1-methyl-1,2,3,6-tetrahydro-4-pyridinyl)indole;
6-Bromo-1-(4-methoxyphenylsulfonyl)-3-(1-methyl-1,2,3,6-tetrahydro-4-pyridinyl)Indole;
6-Bromo-1-(4-fluorophenylsulfonyl)-3-(1-methyl-1,2,3,6-tatrahydro-4-pyridinyl)indole;
5-Cyclohexyloxy-3-(1-methyl-1,2,3,6-tetrahydro-4-pyridinyl)-1-(1-naphthylsulfonyl)indole;
5-Cyclohexyloxy-3-(1-methyl-1,2,3,6-tetrahydro-4-pyridinyl)-1-(2-naphthylsulfonyl)indole;
1-(2-Bromophenylsulfonyl)-5-cyclohexyloxy 3-(1-methyl-1,2,3,6 tetrahydro-4-pyridinyl)indole;
5-Cyclohexyloxy-(1-methyl-1,2,3,6-tetrahydro-4-pyredinyl)-1-(3-nitrophenylsulfonyl)indole;
5-Cyclohexyloxy-3-(1-methyl-1,2,3,6-tetrahydro-4-pyridinyl)-1-(3-nitro-4-trifluoromethylphenylsulfonyl)indole;
1-(4-Bromophenylsulfonyl)-5-cyclohexyloxy-3-(1-methyl-1,2,3,6-tetrahydro-4-pyridinyl)indole;
5-Cyclohexyloxy-1-(4-methoxyphenylsulfony)-3-(1-methyl-1,2,3,6-tetrahydro-4-pyridinyl)indole;
5-Cyclohexyloxy-1-(4-fluorophenylsulfonyl)-3-(1-methyl-1,2,3,6-tetrahydro-4-pyridinyl)indole:
1-(4-Chlorophenylsulfonyl)-5-cyclohexyloxy-3-(1-methyl-1,2,3,6-tetrahydro-4-pyridinyl)indole;
1-(4-t-Butylphenylsulfonyl)-5-cyclohexyloxy-3-(1-methyl-1,2,3,6-tetrahydro-4-pyridinyl)indole;
1-(2-Bromophenylsulfonyl)-5-fluoro-3-(1-methyl-1,2,3,6-tetrahydro-4-pyridinyl)indole;
1-(4-Fluorophenylsulfonyl)-5-fluoro-3-(1-methyl-1,2,3,6-tetrahydro-4-pyridinyl)indole;
1-(4-Chlorophenylsulfonyl)-5-fluoro-3-(1-methyl-1,2,3,6-tetrahydro-4-pyridinyl)indole;
1-(4-t-Butylphenylsulfonyl)-4-fluoro-3-(1-methyl-1,2,3,6-tetrahydro-4-pyridinyl)indole;
5-Fluoro-3-(1-methyl-1,2,3,6-tetrahydro-4-pyridinyl)-1-(3-nitrophenylsulfonyl)indole;
5-Fluoro-3-(1-methyl-1,2,3,6-tetrahydro-4-pyridinyl)-1-(1-naphthylsulfonyl)indole;
5-Fluoro-3-(1-methyl-1,2,3,6-tetrahydro-4-pyridinyl)-1-(2-naphthylsulfonyl)indole;
5-Chloro-1-(4-fluorophenylsulfonyl)3-(1-methyl-1,2,3,6-tatrahydro-4-pyridinyl)indole;
1-(4-Fluorophenylsulfonyl)-3-(1-methyl-1,2,3,6-tetrahydro-4-pyridinyl)-5-trifluoromethoxyindole;
1-(4-t-Butylphenylsulfonyl)-3-(1-methyl-1,2,3,6-tetrahydro-4-pyridinyl)-5-trifluoromethoxyindole;
1-(4-Chlorophenylsulfonyl)-3-(1-methyl-1,2,3,6-tetrahydro-4-pyridinyl)-5-trifluromethoxyindole;
1-(4-Methylphenylsulfonyl)-3-(1-methyl-1,2,3,6-tetrahydro-4-pyridinyl)-5-trifluoromethoxyindole;
(4-Methoxyphenylsulfonyl)-3-(1-methyl-1,2,3,6-tetrahydro-4-pyridinyl)-5-trifluoromethoxyindole;
1-(4-Fluorophenylsulfonyl)-3-(1-methyl-1,2,3,6-tetrahydro-4-pyridinyl)-5-trifluoromethylindole hydrochloride;
1-(4-t-Butylphenylsulfonyl)-3-(1-methyl-1,2,3,6-tetrahydro-4-pyridinyl)-5-trifluoromethylindole hydrochloride;
1-(4-Chlorophenylsulfonyl)-3-(1-methyl-1,2,3,6-tetrahydro-4-pyridinyl)-5-trifluoromethylindole hydrochloride;
1-(4-Methoxyphenylsulfonyl)-3-(1-methyl-1,2,3,6-tetrahydro-4-pyridinyl)-5-trifluoromethylindole hydrochloride;
1-(4-Methylphenylsulfonyl)-3-(1-methyl-1,2,3,6-tetrahydro-4-pyridinyl)-5-trifluoromethylindole hydrochloride;
5-Chloro-1-(4-fluorophenylsulfonyl)-3-(1-methyl-4-piperidinyl)indole;
1-(4-Fluorophenylsulfonyl)-3-(1-methyl-4-piperidinyl)indole;
1-(4-Fluorophenylsulfonyl)-6-chloro-3-(1-methyl-4-piperidinyl)indole;
5,7-Difluoro-3-(1-methyl-1,2,3,6-tetrahydro-4-pyridinyl)-1-(4-methylphenylsulfonyl)indole;
5,7 -Difluoro-1-(4-fluorophenylsulfonyl)-3-(1-methyl-1,2,3 ,6-tetrahydro-4-pyridinyl)indole;
1-(4-Chlorophenylsulfonyl)-5,7-difluoro-3-(1-methyl-1,2,3,6-tetrahydro-4-pyridinyl)indole:
5,7-Difluoro-3-(1-methyl-1,2,3,6-tetrahydro-4-pyridinyl)-1-(1-naphthylsulfonyl)indole;
5,7-Difluoro-3-(1-methyl-1,2,3,6-tetrahydro-4-pyridlnyl)-1-(2-naphthylsulfonyl)indole; and
5,7-Difluoro-3-(1-methyl-1,2,3,6-tetrahydro-4-pyridinyl)-1-(2,4,6-trimethylphenyl)sulfonylindole.

In more specific embodiments of the invention, the compounds of Formula I include:
5-Fluoro-1-(4-methylphenylsulfonyl)-3-(1-methyl-1,2,3,6-tetrahydro-4-pyridinyl)indole;
1-(4-Methoxyphenylsulfonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridinyl)indole;
1-(4-Bromophenylsulfonyl)-5-fluoro-3-(1-methyl-1,2,3,6-tetrahydro-4-pyridinyl)indole;
5-Fluoro-1-(2,5-dichlorophenylsulfonyl)-3-(1-methyl-1,2,3,6-tetrahydro-4-pyridinyl)indole;
1-(4-Chloro-3-nitrophenylsulfonyl)-5-fluro-3-(1-methyl-1,2,3,6-tetrahydro-4-pyridinyl)indole;
5-Fluoro-(1-methyl-1,2,3,6-tetrahydro-4-pyridinyl)-1-(2,4,6-trimethylphenylsulfonyl)indole;
5-Cyclohexyloxy-1-(4-methylphenylsulfonyl)-3-(1-methyl-1,2,3,6-tetrahydro-4-pyridinyl)indole;
6-Bromo-1-(4-fluorophenylsulfonyl)-3-(1-methyl-1,2,3,6-tetrahydro-4-pyridinyl)indole;
5-Cyclohexyloxy-3-(1-methyl-1,2,3,6,tetrahydro-4-pyridinyl)-1-(1-naphthylsulfonyl)Indole;
5-Cyclohexyloxy-3-(1-methyl-1,2,3,6-tetrahydro-4-pyridinyl-(2-naphthylsulfonyl)indole;
1-(2-Bromophenylsulfonyl)-5-cyclohexyloxy-3-(1-methyl)-1,2,3,6-tetrahydro-4-pyridinyl)indole;
5-Cyclohexyloxy-1-(4-methoxyphenylsulfonyl)-3-(1-methyl-1,2,3,6-tetrahydro-4-pyridinyl)indole;
1-(2-Bromophenylsulfonyl)-5-fluoro-3-(1-methyl-1,2,3,6-tetrahydro-4-pyridinyl)Indole
1-(4-Fluorophenylsulfonyl)-5-fluoro-3-(1-methyl-1,2,3,6-tetrahydro-4-pyridinyl)indole;
1-(4-Chlorophenylsulfonyl)-5-fluoro-3-(1-methyl-1,2,3,6 tetrahydro-4-pyridinyl)indole:
5-Fluoro-3-{1-methyl-1,2,3,6-tetrahydro-4-pyridinyl)-1-(3-nitrophanylsulfonyl)indole;
5-Fluoro-3-(1-methyl-1,2,3,6-tetrahydro-4-pyridinyl)-1-(1-naphthylsulfonyl)indole;
5-Fluoro-3-(1-methyl-1,2,3,6-tetrahydro-4-pyridinyl)-1-(2-naphthylsulfonyl)indole;
5-Chloro-1-(4-fluorophenylsulfonyl)-3-(1-methyl-1,2,3,6-tetrahydro-4-pyridinyl)indole;
1-(4-Chlorophenylsulfonyl)-3-(1-methyl-1,2,3,6-tetrahydro-4-pyridinyl)-5-trifluoromethoxyindole;
1-(4-Methylphenylsulfonyl)-3-(1-methyl-1,2,3,6-tetrahydro-4-pyridinyl)-5-trifluoromethoxyindole;
1-(4-Chlorophenylsulfonyl)-3-(1-methyl-1,2,3,6-tetrahydro-4-pyridinyl)-5-trifluoromethylindole hydrochloride:
1-(4-Methoxyphenylsulfonyl)-3-(1-methyl-1,2,3,6-tetrahydro-4-pyridinyl)-5-trifluoromethylindole hydrochloride;
1-(4-Methylphenylsulfonyl)-3-(1-methyl-1,2,3,6-tetrahydro-4-pyridinyl)-5-trifluoromethylindole hydrochloride;
5, 7-Difluoro-3-(1-methyl-1,2,3,6-tetrahydro-4-pyridinyl)-1-(4-methylphenylsulfonyl)indole;
1-(4-Bromophenylsulfonyl)-5,7-difluoro-3-(1-methyl-1,2,3,6-tetrahydro-4-pyridinyl)indole;
1-(4-Chlorophenylsulfonyl)-5,7-difluoro-3-(1-methyl-1,2,3,6-tetrahydro-4-pyridinyl)indole;
5,7-Difluoro-3-(1-methyl-1,2,3,6-tetrahydro-4-pyridinyl)-1-(1-naphthylsulfonyl)indole; and
5,7-Difluoro-3-(1-methyl-1,2,3,6-tetrahydro-4-pyridinyl)-1-(2-naphthylsulfonyl)indole.

In the most specific embodiments of the invention, the compounds of Formula I include:
5-Fluoro-1-(4-methylphenylsulfonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridinyl)indole;
1-(2-Bromophenylsulfonyl)-5-fluoro-3-(1-methyl-1,2,3,6-tetrahydro-4-pyridinyl)indole;
1-(4-Fluorophenylsulfonyl)-5-fluoro-3-(1-methyl-1,2,3,6-tetrahydro-4-pyridinyl)indole;
1-(4-Chlorophenylsulfonyl)-5-fluoro-3-(1-methyl-1,2,3,6-tetrahydro-4-pyridinyl)indole;
5-Fluoro-3-(1-methyl-1,2,3,6-tetrahydro-4-pyridinyl)-1-(1-naphthylsulfonyl)indole;
5-Chloro-1-(4-fluorophenylsulfonyl)-3-(1-methyl-1,2,3,6-tetrahydro-4-pyridinyl)indole;
1-(4-Chlorophenylsulfonyl)-3-(1-methyl-1,2,3,6-tetrahydro-4-pyridinyl)-5-trifluoromethoxyindole;
1-(4-Methylphenylsulfonyl)-3-(1-methyl-1,2,3,6-tetrahydro-4-pyridinyl)-5-trifluoromethoxyindole;
5,7-Difluoro-3-(1-methyl-1,2,3,6-tetrahydro-4-pyridinyl)-1-(4-methylphenylsulfonyl)indole;
1-(4-Bromophenylsulfonyl)-5,7-difluoro-3-(1-methyl-1,2,3,6-tetrahydro-4-pyridinyl)indole;
1-(4-Chlorophenylsulfonyl)-5,7-difluoro-3-(1-methyl-1,2,3,6-tetrahydro-4-pyridinyl)indole;
5,7-Difluoro-3-(1-methyl-1,2,3,6-tetrahydro-4-pyridinyl)-1-(1-naphthylsulfonyl)indole; and
5,7-Difluoro-3-(1-methyl-1,2,3,6-tetrahydro-4-pyridinyl)-1-(2-naphthylsulfonyl)indole.

Acid addition salts of the compounds of Formula I are most suitably formed from pharmaceutically acceptable adds, and include for example those formed with inorganic acids e.g. hydrochloric, sulfuric or phosphoric acids and organic acids e.g. succinic, maleic, acetic or fumaric add. Other non-pharmaceutically acceptable salts e.g. oxalates may be used for example in the isolation of compounds of Formula I for laboratory use, or for subsequent conversion to a pharmaceutically acceptable acid addition salt. Also included within the scope of the invention are solvates and hydrates of the invention.

The conversion of a given compound salt to a desired compound salt is achieved by applying standard techniques, in which an aqueous solution of the given salt is treated with a solution of base e.g. sodium carbonate or potassium hydroxide, to liberate the free base which is then extracted into an appropriate solvent, such as ether. The free base is then separated from the aqueous portion, dried, and treated with the requisite acid to give the desired salt.

Compounds of the present invention may have within its structure a chiral centre. The invention extends to cover all structural and optical isomers of the various compounds, as well as racemic mixtures thereof.

In accordance with other aspects of the invention, the compounds of the present invention can be prepared by processes analogous to those established in the art. For example, as shown in Scheme 1, compounds of Formula I may be prepared by first treating compounds of Formula A, wherein R¹ is C₁₋₄alkyl and R² and R^{4a-d} are as defined in Formula I, with an appropriate base, followed by the addition of a reagent of Formula C which provides a compound of Formula I wherein R³ is SO₂R⁵. Therefore, for example, treatment of compounds of Formula A with a strong base such as lithium diisopropylamide, n-butyllithium or sodium bis(trimethylsilyl)amide in an inert solvent such as tetrahydrofuran or hexanes at a temperature in the range of -100 to 0 °C or, alternatively an organic amine in the presence of 4-dimethylaminopyridine (DMAP), in an inert solvent such as methylene chloride or chloroform, at a temperature in the range of 0-60 °C, followed by the addition of sulfonyl chlorides of Formula C, wherein R⁵ is as defined in Formula I, provides compounds of Formula I wherein R³ is COR⁵. Preferred conditions are sodium bis(trimethylsilyl)amide in tetrahydrofuran at -78 °C followed by warming to room temperature or triethylamine and DMAP in methylene chloride at room temperature. Reagents C are commercially available or can be prepared using standard methods known to those skilled in the art. The preparation of compounds of Formula A is described below.

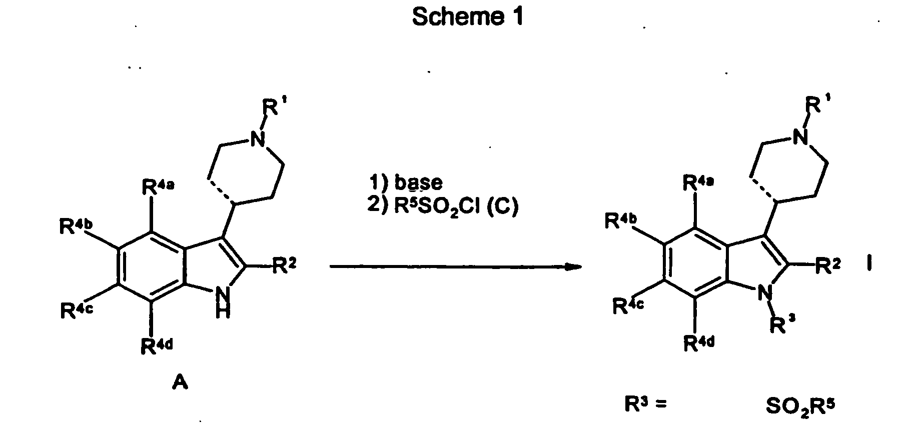

Compounds of Formula I wherein R¹ is H may be prepared by treating a compound of Formula A, wherein R¹ is a suitable protecting group such as t-butoxycarbonyl (t-BOC), with reagents of Formula C as described above. Removal of the protecting group may be performed under standard conditions, for example using acidic conditions such as HCl in ethyl acetate to remove the t-BOC group, to provide compounds of Formula I wherein R¹ is H.

Compounds of Formula I, wherein R² is selected from C₁₋₄alkyl and benzyl, R¹ is C₁₋₄alkyl, R³ is SO₂R⁵ and R^{4a-d} are as defined in Formula I, may also be prepared by treating compounds of Formula 1, wherein R² is H, R¹ is selected from C₁₋₄alkyl, R³ is SO₂R⁵ and R^{4a-d} are as defined in Formula I, with a strong base, such as n-butyllithium, in an inert solvent, such as tetrahydrofuran, at a temperature in the range of -100-0 °C (preferably -78 °C), followed by the addition of a reagent of formula R²-X, wherein R² is selected from C₁₋₄alkyl and benzyl and X is a suitable leaving group such as bromo, followed by warming to room temperature. The above reaction may also be carried out on a compound of Formula I, wherein R² is H, R¹ is a suitable protecting group, such as t-butoxycarbonyl, R³ is SO₂R⁵ and R^{4a-d} are as defined in Formula I, which, after removal of the protecting group using standard deprotection conditions (for example HCl in ethyl acetate to remove the t-butoxycarbonyl protecting group), provides a compound of Formula I wherein R² is selected from C₁₋₄alkyl and benzyl, R¹ is H, R³ is SO₂R⁵ and R^{4a-c} are as defined in Formula I.

Compounds of Formula A wherein R¹, R² and R^{4a-d} are as defined in Formula I may be prepared as shown in Scheme 2. Indole D, wherein R² and R^{4a-d} are as defined in Formula I, may be condensed with a reagent of Formula E, wherein R¹ is as defined in Formula I, in the presence of a base in a suitable solvent at temperatures in the range of 25-100 °C, preferably, 60-90 °C to provide compounds of Formula A wherein R¹, R², R^{4a-d} are as defined in Formula I and ---- represents a double bond. Suitable bases include organic amines such as pyrrolidine or triethylamine and suitable solvents include methanol, ethanol and the like. Preferred conditions are pyrrolidine in ethanol at a refluxing temperature. Compounds of Formula A, wherein R¹, R², R^{4a-d} are as defined in Formula I and ---- represents a double bond, may be reduced using standard hydrogenation conditions or using metal hydride reducing reagents to provide compounds of Formula A where R¹, R², R^{4a-d} are as defined in Formula I and ---- represents a single bond as shown in Scheme 2. Preferred is reduction by hydrogenation, using a suitable catalyst such as palladium or platinum on carbon in methanol at room temperature.

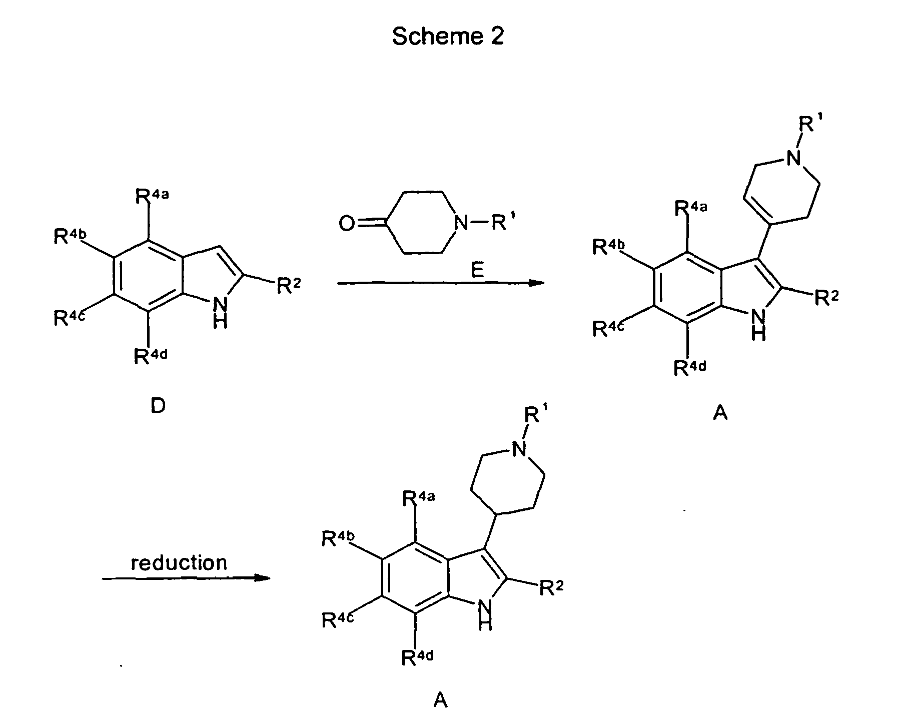

Reagents of Formula A, wherein R¹ is a protecting group and R² and R^{4a-d} are as defined in Formula I, are available by treating reagents of Formula A, wherein R¹ is H, R² and R^{4a-d} are as defined in Formula I and represents a single or double bond, under standard conditions to introduce a protecting group on the piperidine or tetrahydropyridine nitrogen. For example, reaction of indole A, wherein R¹ is H, in the presence of di-t-butyldicarbonate and a base, such as sodium hydroxide, would provide compounds of Formula A wherein R¹ is the t-BOC protecting group, R² and R ^{4a-d} are as defined in Formula I and --- represents a single or double bond.

The indoles of Formula D are either commercially available or can be prepared using standard procedures. For example, compounds of Formula D may be prepared as shown in Scheme 3. 4-Substituted anilines of Formula E, wherein R^{4a-d} are as defined in Formula I, can be treated with reagents of Formula F, wherein R² is as defined in Formula I, in the presence of a base such as sodium bicarbonate or potassium carbonate in an alcoholic solvent at temperatures in the range of 60-100 °C, to provide intermediates of Formula G. Preferred conditions are sodium bicarbonate in ethanol at around 80 °C. Intermediates of Formula G can be cyclized in the presence of reagents of Formula H, wherein R is, for example, methyl or trifluoromethyl (which is preferred) at temperatures in the range of 60-100 °C, to provide indoles of Formula J. The preferred conditions are trifluoroacetic anhydride and trifluoroacetic acid at refluxing temperatures. Finally, compounds of Formula J can be treated under standard deprotection conditions, for example alkali hydroxides in an alcoholic solvent, to provide indoles of Formula D, wherein R² and R^{4a-d} are as defined in Formula I. Preferred conditions for this reaction are potassium hydroxide in ethanol at room temperature. The reagents of Formula E and F, are either commercially available or can be prepared using processes analogous to those established in the art.

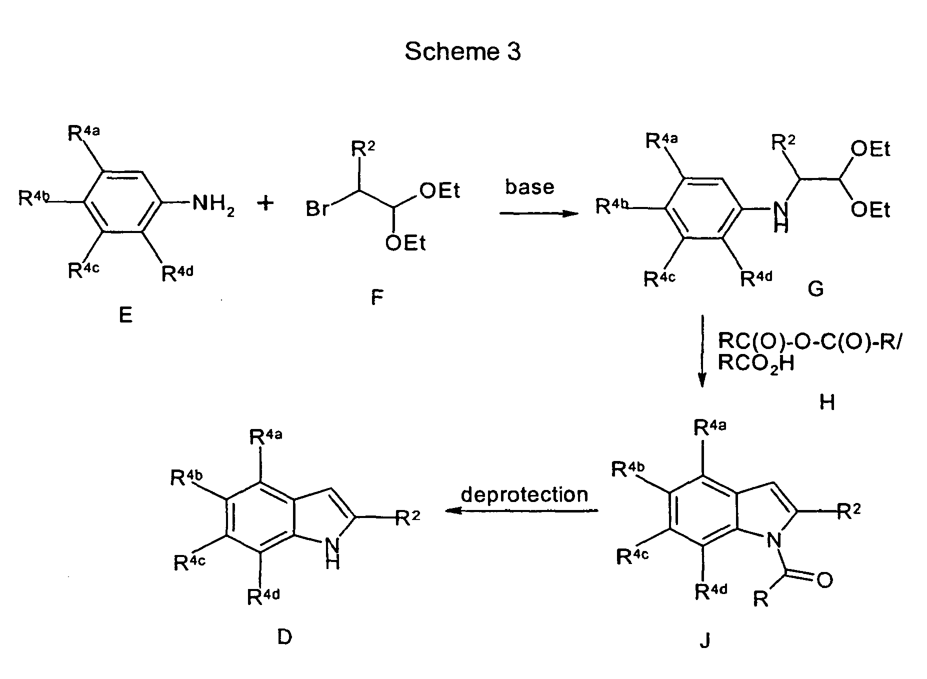

An alternative procedure for preparing indoles of Formula D wherein R^{4b} is vinyl or ethyl is shown in Scheme 4. Indoles of Formula K, wherein Y is a suitable leaving group such as halo or triflate (preferably bromo) and R² is as defined in Formula I, can be coupled with a vinyl trialkylstannane of, for example, Formula L, under standard palladium-cross coupling conditions to provide indoles of Formula D, wherein R^{4b} is vinyl and R² is as defined in Formula I. It will be appreciated that other metal coupling reagents could be used in place of the vinyl stannane, for example, a vinyl boronic acid, chloro zinc and the like. Preferred coupling conditions include heating the indole and vinyl metal, reagent in an inert solvent such as dimethylformamide or toluene in the presence of tetrakis(triphenylphosphine) palladium (0) at refluxing temperatures. Following the coupling reaction, the double bond of the vinyl group can be hydrogenated using catalytic amounts of palladium on carbon in an inert solvent (preferably ethyl acetate) in a hydrogen atmosphere at room temperature to provide indoles of Formula D wherein R^{4b} is ethyl. This procedure could also be used to prepare compounds of Formula D wherein R^{4a}, R^{4c} or R^{4d} are ethyl by reacting the appropriately substituted indole L as described above.

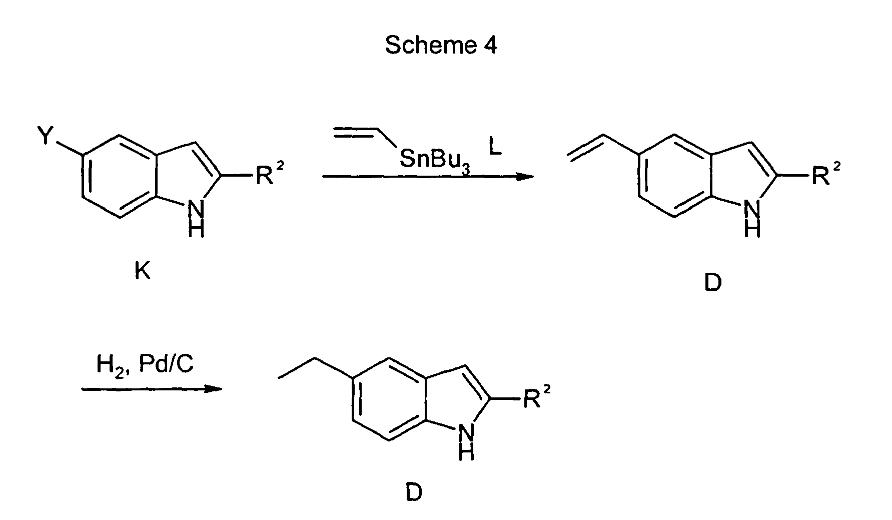

Indoles of Formula D, wherein R^{4b} is cyclooxy or phenoxy and R² is as defined in Formula I, are also available from the corresponding 5-hydroxyindole M as shown in Scheme 5. Reaction of indole M with, for example, cyclohexanol N under standard Mitsunobu conditions (Mitsunobu, O. Synthesis, 1981:1-28) provides reagents of Formula D, wherein R^{4b} is cyclohexyloxy and R² is as defined as Formula I. Reaction of indole M with reagent of Formula O wherein Y is an appropriate leaving group such as halo, preferably iodo, under standard Ulman conditions (Fanta, F. E., Chem. Rev., 64, 1964:613) provides indoles of Formula D, wherein R^{4b} is phenoxy and R² is as defined in Formula I. Preferred conditions are iodobenzene in the presence of potassium carbonate, copper (I) bromide and copper powder in N-methylpyrrolidine at 170 °C.

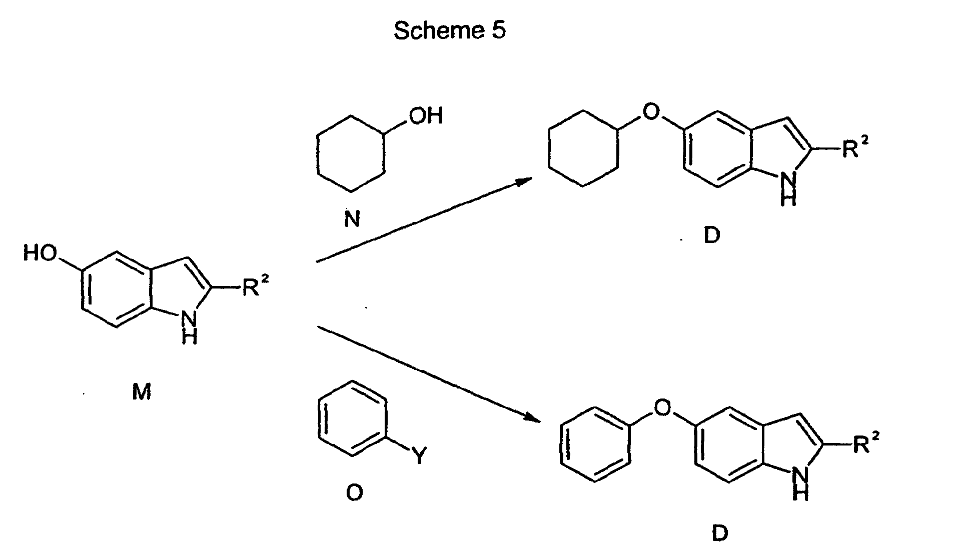

The reactions shown in Schemes 4 and 5 could also be applied to indoles with the 3-position group in place. In some cases, the chemistries outlined above may have to be modified, for instance by use of protecting groups, to prevent side reactions due to reactive groups, such as reactive groups attached as substituents.

The present compounds can be used to distinguish 5-HT₆ receptors from other receptor subtypes, for example glutamate or opioid receptors, within a population of receptors, and in particular to distinguish between the 5-HT₆ and other 5-HT receptor subtypes. The latter can be achieved by incubating preparations of the 5-HT₆ receptor and one of the other 5-HT receptor subtypes (for example 5-HT_{2A}) with a 5-HT₆-selective compound of the invention and then incubating the resulting preparation with a radiolabeled serotonin receptor ligand, for example [³H]-serotonin. The 5-HT₆ receptors are then distinguished by determining the difference in membrane-bound activity, with the 5-HT₆ receptor exhibiting lesser radioactivity, i.e., lesser [³H]-serotonin binding, than the other 5-HT receptor subtype.

The compound may be provided in labeled form, such as radiolabeled form, e. g. labeled by incorporation within its structure ³H or ¹⁴C or by conjugation to ¹²⁵I. The compounds in labeled form can be used to identify 5-HT₆ receptor ligands by techniques common in the art. This can be achieved by incubating the receptor or tissue in the presence of a ligand candidate and then incubating the resulting preparation with an equimolar amount of radiolabeled compound of the invention such as [³H]-5-fluoro-1-(4-methylphenylsulfonyl)-3-(1-methyl-1,2,3,6-tetrahydro-4-pyridinyl)indole. 5-HT₆ receptor ligands are thus revealed as those that are not significantly displaced by the radiolabeled compound of the present invention. Alternatively, 5-HT₆ receptor ligand candidates may be identified by first incubating a radiolabeled form of a compound of the invention then incubating the resulting preparation in the presence of the candidate ligand. A more potent 5-HT₆ receptor ligand will, at equimolar concentration, displace the radiolabeled compound of the invention.

The present compounds are useful as pharmaceuticals for the treatment of various conditions in which the use of a 5-HT₆ antagonist is indicated, such as psychosis, schizophrenia, manic depression, depression, neurological disturbances, memory disturbances, Parkinsonism, amylotrophic lateral sclerosis, Alzheimer's disease and Huntington's disease. In another of its aspects, the present invention provides pharmaceutical compositions useful to treat 5-HT₆-related medical conditions, in which a compound of Formula I is present in an amount effective to antagonize 5-HT₆ receptor stimulation, together with a pharmaceutically acceptable carrier. The compounds are useful for treating medical conditions for which a 5-HT₆ receptor antagonist is indicated, which comprises the step of administering to the patient an amount of a compound of Formula I effective to antagonize 5-HT₆ receptor stimulation, an a pharmaceutically acceptable carrier therefor.

For use in medicine, the compounds of the present invention can be administered in a standard pharmaceutical composition. The present invention therefore provides, in a further aspect, pharmaceutical compositions comprising a pharmaceutically acceptable carrier and a Formula I compound or a pharmaceutically acceptable salt, solvate or hydrate thereof, in an amount effective to treat the target indication.

The compounds of the present invention may be administered by any convenient route, for example by oral, parenteral, buccal, sublingual, nasal, rectal, patch, pump or transdermal administration and the pharmaceutical compositions formulated accordingly.

Compounds of Formula I and their pharmaceutically acceptable salts which are active when given orally can be formulated as liquids, for example syrups, suspensions or emulsions, or as solid forms such as tablets, capsules and lozenges. A liquid formulation will generally consist of a suspension or solution of the compound or pharmaceutically acceptable salt in a suitable pharmaceutical liquid carrier for example, ethanol, glycerine, non-aqueous solvent, for example polyethylene glycol, oils, or water with a suspending agent, preservative, flavouring or colouring agent. A composition in the form of a tablet can be prepared using any suitable pharmaceutical carrier routinely used for preparing solid formulations. Examples of such carriers include magnesium stearate, starch, lactose, sucrose and cellulose. A composition in the form of a capsule can be prepared using routine encapsulation procedures. For example, pellets containing the active ingredient can be prepared using standard carriers and then filled into hard gelatin capsule; alternatively, a dispersion or suspension can be prepared using any suitable pharmaceutical carrier, for example aqueous gums, celluloses, silicates or oils and the dispersion or suspension filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilized and then reconstituted with a suitable solvent just prior to administration.

Compositions for nasal administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomising device. Alternatively, the sealed container may be a unitary dispensing device such as a single dose nasal inhaler or an aerosol dispenser fitted with a metering valve which is intended for disposal after use. Where the dosage form comprises an aerosol dispenser, it will contain a propellant which can be a compressed gas such as compressed air or an organic propellant such as fluorochlorohydrocarbon. The aerosol dosage forms can also take the form of a pump-atomizer.

Compositions suitable for buccal or sublingual administration include tablets, lozenges, and pastilles, wherein the active ingredient is formulated with a carrier such as sugar, acacia, tragacanth, or gelatin and glycerine. Compositions for rectal administration are conveniently in the form of suppositories containing a conventional suppository base such as cocoa butter.

Preferably, the composition is in unit dose form such as a tablet, capsule or ampoule. Suitable unit dosages, i.e. therapeutically effective amounts, can be determined during clinical trials designed appropriately for each of the conditions for which administration of a chosen compound is indicated and will of course vary depending on the desired clinical endpoint. Each dosage unit for oral administration may contain from 0.01 to 500 mg/kg (and for parenteral administration may contain from 0.1 to 50 mg) of a compound of Formula I, or a pharmaceutically acceptable salt thereof calculated as the free base, and will be administered in a frequency appropriate for initial and maintenance treatments. For laboratory use, the present compounds can be stored in packaged form for reconstitution and use.

### Experimental Examples

### Information Example 1: 5-Cyclohexyloxy-1 H-indole:

Triphenylphosphine (10.3 g, 39.4 mmol) and 5-hydroxy-1 H-indole (5 g, 36.9 mmol) were added to a solution of cyclohexanol (3.7 mL, 35.7 mmol) in THF (200 mL) at 0 °C. DEAD (5.9 mL, 39.4 mmol) was slowly added and the resulting solution was stirred for 1 week at room temperature. The solvent was removed *in vacuo* and flash chromatography (silica gel, 10% ethyl acetate in hexane) yielded 5-cyclohexyloxy-1H-indole (3.2 g, 40%).

### Information Example 2: 5-Trifluoromethoxy-1H-indole

Lithium aluminum hydride (10.4 mL,1M in THF, 10.4 mmol) was added to a solution of 5-trifluoromethoxyistatin (0.8 g, 3.45 mmol) in THF at room temperature and the mixture was refluxed overnight. The reaction mixture was cooled, and Na₂SO₄·10H₂O was added very carefully portionwise, followed by ethyl acetate. The reaction mixture was then filtered, and the filtrate was evaporated. The residual oil was purifed by column chromatography with ethyl acetate in hexanes (2:98) to yield 5-trifluoromethoxy-1H-indole (0.162 g, 23%).

### Information Example 3: 5,7-Difluoro-1 H-indole

Vinylmagnesium bromide (30 mL, 1 M in THF, 30 mmol) was added dropwise to a solution of 2,4-difluoronitrobenzene (1.6 g, 10 mmol) in THF at - 40 °C.

The mixture was stirred for 30 min and then poured into CH₂Cl₂-diluted HCl. The organic layer was separated and dried (Na₂SO₄). After the solvent was removed *in vacuo,* the residual oil was purifed by column chromatography with EtOAc/Hexanes (2:98) to yield 2,4-difluoro-1H-indole (189 mg, 12%).

### Example 1(a): 5-Bromo-3-(1-methyl-1,2,3,6-tetrahydro-4-pyridinyl)-1 H-indole

5-Bromo-1H-indole (4.31 g, 22 mmol), 1-methyl-4-piperidone (2.46 mL, 20 mmol) and pyrrolidine (17 mL, 200 mmol) were mixed in ethanol (30 mL) and refluxed for 72 hours. The mixture was cooled to room temperature and the resulting solid, collected by filtration, washed with methanol and dried to provide the title compound as a white solid (4.40 g, 76%). mp >230 °C, dec.

In a like manner, the following additional compounds were prepared:
(b) 5-Fluoro-3-(1-methyl-1,2,3,6-tetrahydro-4-pyridinyl)-1H-indole: from 5-fluoro-1 H-indole;
(c) 5-Cyclohexyloxy-3-(1-methyl-1,2,3,6-tetrahydro-4-pyridinyl)-1H-indole: from 5-cyclohexyloxy 1H-indole (Example 1);
(d) 5-Chloro-3-(1-methyl-1,2,3,6-tetrahydro-4-pyridinyl)-1H-indole: from 5-chloro-1 H-indole;
(e) 3-(1-methyl-1,2,3,6-tetrahydro-4-pyridinyly)-1H-indole: from 1 H-indole;
(f) 6-Bromo-3-(1-methyl-1,2,3,6-tetrahydro-4-pyridinyl)-1H-indole: from 6-bromo-1 H-indole;
(g)5,7-Difluoro-3-(1-methyl-1,2,3,6-tetrahydro-4-pyridinyl)-1H-indole: (176.8 mg, 58%); from 5,7-difluoroindole (Information Example 3, 188 mg, 1.23 mmol) and N-methyl-4-piperidone (153.4 mg, 1.23 mmol) with pyrrolidine (875 mg, 12.3 mmol) in ethanol (1.5 mL) at reflux. m.p 215-21 °C, HRMS-FAB⁺ for C₁₄H₁₄N₂F₂: calc.MH⁺: 249.12033 ; found:249.12095;
(h) 3-(1-Methyl-1,2,3,6-tetrahydro-4-pyridinyl)-5-trifluoromethoxy-1 H-indole: from 5-trifluoromethoxy-1H-indole (Example 2);
(i) 3-(1-Methyl-1,2,3,6-tetrahydro-4-pyridinyl)-5-trifluoromethyl-1 H-indole: from 5-trifluoromethyl-1 H-indole (prepared according to Miyano, et al., JP-60204759);
(j) 6-Chloro-3-(1-methyl-1,2,3,6-tetrahydro-4-pyridinyl)-1H-indole: from 6-chloro-1 H-indole.

### Reference Example 2(a): 5-Cyclohexyloxy-3-(1-methyl-4-piperidinyl)-1 H-indole

Palladium on carbon (10%. 31 mg) was added to a solution of 5-cyclohexyloxy-3-(1-methyl-1,2,3,6-tetrahydro-4-pyridinyl)-1H-indole (Example 1c, 48.4 mg, 0.16 mmol) in methanol (5 mL) and the mixture was stirred under an atmosphere of hydrogen for 8h. Removal of the catalyst by filtration through celite and silica (20% 2M methanolic ammonia in dichloromethane) yielded 5-cyclohexyloxy-3-(1 -methyl-4-piperidinyl)-1 H-indole (quantitative, HRMS-FAB⁺ for C₂₀H₂₈N₂O: calculated MH⁺:313.22800; found MH⁺:313.23083).

In a like manner, the following additional compounds were prepared:
(b) 6-Chloro-3-(1-methyl-4-piperidinyl)-1H-indole: (0.815 g, 81%); from 6-chloro-3-(1-methyl-1,2,3,6-tetrahydro-4-pyridinyl)-1H-indole (Example 1j, 1.0 g, 4 mmol) and 5% Pt/C (0.78 g, 0.2 mmol) in ethanol under H₂; m.pt. 218-24°C.
(c) 5-Fluoro-3-(1-methyl-4-piperidinyl)-1H-indole: (0.64 g, 64%); from 5-fluoro-3-(1-methyl-1,2,3,6-tetrahydro-4-pyridinyl)-1H-indole (Example 1b, 1.0 g, 4.34 mmol) and 5% Pt/C(0.43 g, 0.4 mmol) in ethanol under H₂; mp 158-61°C. HRMS-FAB⁺ for C₁₄H₁₇N₂F caculated MH⁺: 233.14540, found: 233.13193.
(d) 3-(1-Methyl-4-piperidinyl)-1H-indole: (0.92 g, 91%); from 3-(1-methyl-1,2,3,6-tetrahydro-4-pyridinyl)-1H-indole (Example 1e, 1.0 g,4.69 mmol) and 10% Pd/C on carbon (0.5 g, 0.47 mmol) in ethanol under H₂; mp 174-6°C; HRMS-FAB⁺ for C₁₄H₁₈N₂, caculated MH⁺: 215.15483, found: 215.15356.
(e) 5-Chloro-3-(1-methyl-4-piperidinyl)indole: (0.89 g, 88%); from 5-chloro-3-(1-methyl-1,2,3,6-tetrahydro-4-pyridinyl)indole (Example 1d, 1.0 g , 4 mmol) and 5% Pt/C (0.78 g, 0.2mmol) in methanol under H₂; mp 192-4°C; HRMS-FAB⁺ for C₁₄H₁₇N₂Cl calculated MH⁺: 249.11584, found: 249.11529.

### Reference Example 3(a): 1-Benzoyl-5-fluoro-3-(1-methyl-1,2,3,6-tetrahydro-4-pyridinyl)indole

Benzoyl chloride (33.3 mg, 0.24 mmol) was added to a solution of 5-fluoro-3-(1-methyl-1,2,3,6-tetrahydro-4-pyridinyl)indole (Example 1b, 49.6 mg. 0.215 mmol), triethylamine (0.15 mL, 1.1 mmol) and DMAP (5 mg) in dichloromethane (2 mL). The mixture was stirred at room temperature prior to quenching with water and extraction into dichloromethane. The organic layer was washed with brine and dried over sodium sulfate. Purification by flash chromatography (silica gel, 2M methanolic ammonia in chloroform) yielded 1-benzoyl-5-fluoro-3-(1-methyl-1,2,3,6-tetrahydro-4-pyridinyl)indole (27.8 mg, 39%, HRMS-FAB⁺ for C₂₁H₂₀N₂OF calculated MH⁺: 335.15598; found: 335.15457).

In a like manner, the following additional compounds were prepared:
Example 3(b) 5-Fluoro-1-(4-methylphenylsulfonyl)-3-(1-methyl-1,2,3,6-tetrahydro-4-pyridinyl)indole: (7.2 mg, 8%), from 5-fluoro-3-(1-methyl-1,2,3,6-tetrahydro-4-pyridinyl)indole (Example 1b, 52.0 mg, 0.226 mmol) and 4-methylphenylsulfonyl chloride (48.0 mg, 0.25 mmol); HRMS-FAB⁺ for C₂₁H₂₂N₂O₂SF: calculated MH⁺: 385.13861; found: 385.14053.

### Example 4(a): 3-(1-Methyl-1,2,3,6-tetrahydro-4-pyridinyl)-1-phenylsulfonylindole

Sodium bis(trimethylsilyl)amide (0.23 mL, 1M inTHF, 0.23 mmol) was added to a solution of 3-(1-methyl-1,2,3,6-tetrahydro-4-pyridinyl)indole (Example 1e, 25.0 mg, 0.12 mmol) in THF (1.5 mL) at -78°C and the mixture was stirred for 1 h. Phenylsulfonyl chloride (30 uL, 0.24 mmol) was added and the mixture stirred at room temperature for 2h. prior to quenching with water (4 drops) and silica gel (~1g). Purification using solid phase extraction tubes (1000 mg silica, eluting with 0-10% 2M methanolic ammonia in dichloromethane) yielded 3-(1-methyl-1,2,3,6-tetrahydro-4-pyridinyl)-1-phenylsulfonylindole (9.0 mg, 22%. HRMS-FAS⁺ for C₂₀H₂₀N₂O₂S: calculated MH⁺: 353.13239; found: 353.13235).

In a like manner, the following additional compounds were prepared:
(b) 1-(4-Methoxyphenylsulfonyl)-3-(1-methyl-1,2,3,6-tetrahydro-4-pyridinyl)indole: (9.0 mg, 20%); from 3-(1-methyl-1,2,3,6-tetrahydro-4-pyridinyl)-1 H-indole (Example 1e, 24.8 mg, 0.12 mmol) and 4-methoxyphenylsulfonyl chloride (48.2 mg, 0.23 mmol); HRMS-FAB⁺ for C₂₁H₂₂N₂O₃S: calculated MH⁺: 383.14294; found: 383.14276.
(c) 1-(4-Fluorophenylsulfonyl)-3-(1-methyl-1,2,3,6-tetrahydro-4-pyridinyl)indole: (9.0 mg, 21%); from 3-(1-methyl-1,2,3,6-tetrahydro-4-pyridinyl)-1 H-indole (Example 1e, 24.8 mg, 0.12 mmol) and 4-fluorophenylsulfonyl chloride (46.5 mg, 0.24 mmol); HRMS-FAB⁺ for C₂₀H₁₉N₂O₂SF: calculated MH⁺: 371.12296; found: 371.12323.
(d) 1-(4-Bromophenylsulfonyl)-5-fluoro-3-(1-methyl-1,2,3,6-tetrahydro-4-pyridinyl)indole: (43.8 mg, 90%); from 5-fluoro-3-(1-methyl-1,2,3,6-tetrahydro-4-pyridinyl)-1 H-indole (Example 1b, 25.0 mg, 0. 108 mmol) and 4-bromophenylsulfonyl chloride (56.4 mg, 0.22 mmol); HRMS-FAB⁺ for C₂₀H₁₈N₂O₂SFBr: calculated MH⁺: 449.03348; found: 449.03297.
(e) 5-Fluoro-1-(2,5-dichlorophenylsulfonyl)-3-(1-methyl-1,2,3,6-tetrahydro-4-pyridinyl)indole: (36.5 mg, 76%); from 5-fluoro-3-(1-methyl-1,2,3,6-tetrahydro-4-pyridinyl)-1 H-indole (Example 1b, 25.3 mg, 0.110 mmol) and 2,5-dichlorophenylsulfonyl chloride (55.6 mg, 0.23 mmol); HRMS-FAB⁺ for C₂₀H₁₇N₂O₂SFCl₂: calculated MH⁺: 439.04501; found: 439.04294.
(f) 1-(4-Chloro-3-nitrophenylsulfonyl)-5-fluoro-3-(1-methyl-1,2,3,6-tetrahydro-4-pyridinyl)indole: (39.1 mg, 79%); from 5-fluoro-3-(1-methyl-1,2,3,6-tetrahydro-4-pyridinyl)-1H-indole (Example 1b, 25.4 mg, 0.110 mmol) and 4-chloro-3-nitrophenylsulfonyl chloride (57.6 mg, 0.22 mmol); HRMS-FAB⁺ for C₂₀H₁₇N₂O₄SFCl: calculated MH⁺: 450.06906; found: 450.07176.
(g) 5-Fluoro-3-(1-methyl-1,2,3,6-tetrahydro-4-pyridinyl)-1-(2,4,6-trimethylphenylsulfonyl)indole: (34.7 mg, 76%); from 5-fluoro-3-(1-methyl-1,2,3,6-tetrahydro-4-pyridinyl)-1H-indole (Example 1b, 25.5 mg, 0.110 mmol) 2,4,6-trimethylphenylsulfonyl chloride (48.4 mg, 0.22 mmol); HRMS-FAB⁺ for C₂₃H₂₅N₂O₂SF: calculated MH⁺: 413.16989; found: 413.17396.
(h) 5-Fluoro-3-(1-methyl-1,2,3,6-tetrahydro-4-pyridinyl)-1-(2,4,6-triisopropylphenylsulfonyl)indole: (25.8 mg, 48%); from 5-fluoro-3-(1-methyl-1,2,3,6-tetrahydro-4-pyridinyl)-1H-indole (Example 1b, 24.9 mg, 0.108 mmol) 2,4,6-triisopropylphenylsulfonyl chloride (68.8 mg, 0.23 mmol); HRMS-FAB⁺ for C₂₉H₃₇N₂O₂SF: calculated MH⁺: 497.26379; found: 497.26112.
(i) 5-Cyclohexyloxy-1-(4-methylphenylsulfonyl)-3-(1-methyl-1,2,3,6-tetrahydro-4-pyridinyl)indole: (49.0 mg, 94%); from 5-cyclohexyloxy-3-(1-methyl-1,2,3,6-tetrahydro-4-pyridinyl)-1H-indole (Example 1c, 35.0 mg, 0.112 mmol) and 4-methylphenylsulfonyl chloride (41 mg, 0.22 mmol); HRMS-FAB⁺ for C₂₇H₃₂N₂O₃S: calculated MH⁺: 465.22119; found: 465.22309.
(j) 6-Bromo-1-(4-methoxyphenylsulfonyl)-3-(1-methyl-1,2,3,6-tetrahydro-4-pyridinyl)indole: (24.5 mg, 62%); from 6-bromo-3-(1-methyl-1,2,3,6-tetrahydro-4-pyridinyl)1 H-indole (Example 1f, 25.0 mg, 0.086 mmol) and 4-methoxyphenylsulfonyl chloride (35 mg, 0.17 mmol); HRMS-FAB⁺ for C₂₁H₂₁N₂O₃BrS: calculated MH⁺: 461.05344; found: 461.04886.
(k) 6-Bromo-1-(4-fluorophenylsulfonyl)-3-(1 -methyl-1,2,3,6-tetrahydro-4-pyridinyl)-indole: (20.9 mg, 54%); from 6-bromo-3-(1-methyl-1,2,3,6-tetrahydro-4-pyridinyl)-1 H-indole (Example 1f, 25.1 mg, 0.086 mmol) and 4-fluorophenylsulfonyl chloride (35 mg, 0.18 mmol); HRMS-FAB⁺ for C₂₀H₁₈N₂O₂BrSF: calculated MH⁺: 449.03348; found: 449.02907.
(l) 5-Cyclohexyloxy-3-(1-methyl-1,2,3,6-tetrahydro-4-pyridinyl)-1-(1-naphthylsulfonyl)indole: (35.9 mg, 87%); from 5-cyclohexyloxy-3-(1-methyl-1,2,3,6-tetrahydro-4-pyridinyl)-1 H-indole (Example 1c, 25.5 mg, 0.082 mmol) and 1-naphthylsulfonyl chloride (37.7 mg, 0.17 mmol); HRMS-FAB⁺ for C₃₀H₃₂N₂O₃S: calculated MH⁺: 501.22119; found: 501.21807.
(m) 5-Cyclohexyloxy-3-(1-methyl-1,2,3,6-tetrahydro-4-pyridinyl)-1-(2-naphthylsulfonyl)indole: (37.4 mg, 92%); from 5-cyclohexyloxy-3-(1-methyl-1,2,3,6-tetrahydro-4-pyridinyl)-1H-indole (Example 1c, 25.2 mg, 0.081 mmol) and 2-naphthylsulfonyl chloride (38.5 mg, 0.17 mmol); HRMS-FAB⁺ for C₃₀H₃₂N₂O₃S: calculated MH⁺: 501.22119; found: 501.22101.
(n) 1-(2-Bromophenylsulfonyl)-5-cyclohexyloxy-3-(1-methyl-1,2,3,6-tetrahydro-4-pyridinyl)indole: (34.4 mg, 82%); from 5-cyclohexyloxy-3-(1-methyl-1,2,3,6-tetrahydro-4-pyridinyl)-1H-indole (Example 1c, 24.5 mg, 0.079 mmol) and 2-bromophenylsulfonyl chloride (40.2 mg, 0.16 mmol); HRMS-FAB⁺ for C₂₆H₂₉N₂O₃SBr: calculated MH⁺: 529.11603; found: 529.11380.
(o) 5-Cyclohexyloxy-1-(2,5-dichlorophenylsulfonyl)-3-(1-methyl-1,2,3,6-tetrahydro-4-pyridinyl)indole: (37.3 mg, 89%); from 5-cyclohexyloxy-3-(1-methyl-1,2,3,6-tetrahydro-4-pyridinyl)-1H-indole (Example 1c, 25.5 mg, 0.082 mmol) and 2,5-dichlorophenylsulfonyl chloride (42.0 mg, 0.17 mmol); HRMS-FAB⁺ for C₂₆H₂₈N₂O₃SCl₂: calculated MH⁺: 519.12762; found: 519.12579.
(p) 5-Cyclohexyloxy-3-(1-methyl-1,2,3,6-tetrahydro-4-pyridinyl)-1-(2,4,6-trimethylphenylsulfonyl)indole: (31.6 mg, 78%); from 5-cyclohexyloxy-3-(1-methyl-1,2,3,6-tetrahydro-4-pyridinyl)-1H-indole (Example 1c, 25.5 mg, 0.082 mmol) 2,4,6-trimethylphenylsulfonyl chloride (36.8 mg, 0.17 mmol); HRMS-FAB⁺ for C₂₉H₃₈N₂O₃S: calculated MH⁺: 493.25250; found: 493.25329.
(q) 5-Cyclohexyloxy-3-(1-methyl-1,2,3,6-tetrahydro-4-pyridinyl)-1-(2,4,6-triisopropylphenylsulfonyl)indole: (23.3 mg, 50%); from 5-cyclohexyloxy-3-(1-methyl-1,2,3,6-tetrahydro-4-pyridinyl)-1H-indole (Example 1c, 25.2 mg, 0.081 mmol) 2,4,6-triisopropylphenylsulfonyl chloride (53.7 mg, 0.18 mmol); HRMS-FAB⁺ for C₃₅H₄₈N₂O₃S: calculated MH⁺: 577.34637; found: 577.34710.
(r) 5-Cyclohexyloxy-3-(1-methyl-1,2,3,6-tetrahydro-4-pyridinylyl-1-(3-nitrophenylsulfonyl)indole: (38.0 mg, 96%); from 5-cyclohexyloxy-3-(1-methyl-1,2,3,6-tetrahydro-4-pyridinyl)-1H-indole (Example, 1c, 24.8 mg, 0.080 mmol) and 3-nitrophenylsulfonyl chloride (35.3 mg, 0.159 mmol); HRMS-FAB⁺ for C₂₆H₂₉N₃O₅S: calculated MH⁺: 496.19061; found: 496.19042.
(s) 1-(4-Chloro-3-nitrophenylsulfonyl)-5-cyclohexyloxy-3-(1-methyl-1,2,3,6-tetrahydro-4-pyridinyl)indole: (37.4 mg, 87%); from 5-cyclohexyloxy-3-(1-methyl-1,2,3,6-tetrahydro-4-pyridinyl)-1 H-indole (Example 1c, 25.1 mg, 0.081 mmol) and 4-chloro-3-nitrophenylsulfonyl chloride (42.7 mg, 0.17 mmol); HRMS-FAB⁺ for C₂₆H₂₈N₃O₅SCI: calculated MH⁺: 530.15167; found: 530.14727.
(t) 5-Cyclohexyloxy-3-(1-methyl-1,2,3,6-tetrahydro-4-pyridinyl)-1-(3-nitro-4-trifluoromethylphenylsulfonyl)indole: (33.9 mg, 75%); from 5-cyclohexyloxy-3-(1-methyl-1,2,3,6-tetrahydro-4-pyridinyl)-1H-indole (Example 1c, 24.8 mg, 0.080 mmol) and 3-nitro-4-trifluoromethylphenylsulfonyl chloride (48.4 mg, 0.167 mmol); HRMS-FAB⁺ for C₂₇H₂₈N₃O₅SF₃: calculated MH⁺: 564.17798; found: 564.17855.
(u) 1-(4-Bromophenylsulfonyl)-5-cyclohexyloxy-3-(1-methyl-1,2,3,6-tetrahydro-4-pyridinyl)indole: (31.4 mg, 72%); from 5-cyclohexyloxy-3-(1-methyl-1,2,3,6-tetrahydro-4-pyridinyl)-1 H-indole (Example 1c, 25.7 mg, 0.083 mmol) and 4-bromophenylsulfonyl chloride (43 mg, 0.17 mmol); HRMS-FAB⁺ for C₂₆H₂₉N₂O₃SBr: calculated MH⁺: 529.11603; found: 529.11817.
(v) 5-Cyclohexyloxy-1-(4-methoxyphenylsulfonyl)-3-(1-methyl-1,2,3,6-tetrahydro-4-pyridinyl)indole: (34.8 mg, 89%); from 5-cyclohexyloxy-3-(1-methyl-1,2,3,6-tetrahydro-4-pyridinyl)-1H-indole (Example 1c, 25.2 mg, 0.081 mmol) and 4-methoxyphenylsulfonyl chloride (34.1 mg, 0.165 mmol); HRMS-FAB⁺ for C₂₇H₃₂N₂O₄S: calculated MH⁺: 481.21609; found: 481.21625.
(w) 5-Cyclohexyloxy-1-(4-fluorophenylsulfonyl)-3-(1-methyl-1,2,3,6-tetrahydro-4-pyridinyl)indole: (34.0 mg, 88%); from 5-cyclohexyloxy-3-(1-methyl-1,2,3,6-tetrahydro-4-pyridinyl)-1H-indole (Example 1c, 25.5 mg, 0.082 mmol) and 4-fluorophenylsulfonyl chloride (30.8 mg, 0.16 mmol); HRMS-FAB⁺ for C₂₆H₂₉N₂O₃SF: calculated MH⁺: 469.19611; found: 469.19626.
(x) 1-(4-Chlorophenylsulfonyl)-5-cyclohexyloxy-3-(1-methyl-1,2,3,6-tetrahydro-4-pyridinyl)indole: (37.7 mg, 94%); from 5-cyclohexyloxy-3-(1-methyl-1,2,3,6-tetrahydro-4-pyridinyl)-1H-indole (Example 1c, 25.5 mg, 0.082 mmol) and 4-chlorophenylsulfonyl chloride (35.6 mg, 0.169 mmol); HRMS-FAB⁺ for C₂₆H₂₉N₂O₃SCl: calculated MH⁺: 485.16656; found: 485.16480.
(y) 1-(4-t-Butylphenylsulfonyl)-5-cyclohexyloxy-3-(1-methyl-1 ,2,3,6-tetrahydro-4-pyridinyl)indole: (34.0 mg, 81%); from 5-cyclohexyloxy-3-(1-methyl-1,2,3,6-tetrahydro-4-pyridinyl)-1H-indole (Example 1c, 25.6 mg, 0.082 mmol) and 4-t-butylphenylsulfonyl chloride (37.2 mg, 0.16 mmol); HRMS-FAB⁺ for C₃₀H₃₈N₂O₃S: calculated MH⁺: 507.26813; found: 507.27183.
(z) 1-(2-Bromophenylsulfonyl)-5-fluoro-3-(1-methyl-1,2.3,6-tetrahydro-4-pyridinyl)indole: (32 mg, 53%), from 5-fluoro-3-(1-methyl-1,2,3,6-tetrahydro-4-pyridinyl)-1H-indole (Example 1b, 30.6 mg, 0.133 mmol) and 2-bromophenylsulfonyl chloride (67.9 mg, 0.27 mmol); HRMS-FAB⁺ for C₂₀H₁₉N₂SO₂BrF calculated MH⁺: 449.03348; found: 449.03240.
(aa)-(4-Fluorophenylsulfonyl)-5-fluoro-3-(1-methyl-1,2,3,6-tetrahydro-4-pyridinyl)indole: (30 mg, 57%), from 5-fluoro-3-(1-methyl-1,2,3,6-tetrahydro-4-pyridinyl)-1 H-indole (Example 1b) and 4-fluorophenylsulfonyl chloride (52.9 mg, 0.27 mmol); HRMS-FAB⁺ for C₂₀H₁₉N₂O₂SF₂ calculated MH⁺: 389.11353; found: 389.11544.
(bb) 1-(4-Chlorophenylsulfonyl)-5-fluoro-3-(1-methyl-1,2,3,6-tetrahydro-4-pyridinyl)indole: (28.1 mg, 53%), from 5-fluoro-3-(1-methyl-1,2,3,6-tetrahydro-4-pyridinyl)-1H-indole (Example 1b, 30.1 mg, 0.131 mmol) and 4-chlorophenylsulfonyl chloride (55.3 mg, 0.26 mmol); HRMS-FAB⁺ for C₂₀H₁₉N₂O₂SCIF calculated MH⁺: 405.08998; found: 405.08291.
(cc) 1-(4-t-Butylphenylsulfonyl)-5-fluoro-3-(1-methyl-1,2,3,6-tetrahydro-4-pyridinyl)indole: (23.0 mg, 42%), from 5-fluoro-3-(1-methyl-1,2,3,6-tetrahydro-4-pyridinyl)-1H-indole (Example 1b, 29.4 mg, 0.128 mmol) and 4-t-butylphenylsulfonyl chloride (59.6 mg, 0.26 mmol); HRMS-FAB⁺ for C₂₄H₂₈N₂O₂SF calculated MH⁺: 427.18555; found: 427.18680.
(dd) 5-Fluoro-3-(1-methyl-1,2,3,6-tetrahydro-4-pyridnyl)-1-(3-nitrophenylsulfonyl)indole: (31.0 mg, 56%), from 5-fluoro-3-(1-methyl-1,2,3,6-tetrahydro-4-pyridinyl)-1H-indole (Example 1b, 30.1 mg, 0.131 mmol) and 3-nitrophenylsulfonyl chloride (58.1 mg, 0.26 mmol); HRMS-FAB⁺ for C₂₀H₁₉N₃O₄SF calculated MH⁺: 416.10803; found: 416.10631.
(ee) 5-Fluoro-3-(1-methyl-1,2,3,6-tetrahydro-4-pyridinyl)-1-(1-naphthylsulfonyl)indole: (28.2 mg, 52%), from 5-fluoro-3-(1-methyl-1,2,3,6-tetrahydro-4-pyndinyl)-1H-indole (Example 1b, 29.4 mg, 0.128 mmol) and 1-naphthylsulfonyl chloride (58 mg, 0.26 mmol); HRMS-FAB⁺ for C₂₂H₂₂N₂O₂SF calculated MH⁺: 421.13861; found: 421.14099.
(ff) 5-Fluoro-3-(1-methyl-1,2,3,6-tetrahydro-4-pyridinyl)-1-(2-naphthylsulfonyl)indole: (32.3 mg, 56%), from 5-fluoro-3-(1-methyl-1,2,3,6-tetrahydro-4-pyridinyl)-1H-indole (Example 1b, 31.4 mg, 0.136 mmol) and 2-naphthylsulfonyl chloride (61.7 mg, 0.27 mmol); HRMS-FAB⁺ for C₂₄H₂₂N₂O₂SF calculated MH⁺: 421.13861; found: 421.13866.
(gg) 5-Chloro-1 -(4-fluorophenylsulfonyl)-3-(1-methyl-1,2,3,6-tetrahydro-4-pyridinyl)indole: from 5-chloro-3-(1-methyl-1,2,3,6-tetrahydro-4-pyridinyl)-1H-indole (Example 1d, 25 mg, 0.1 mmol) and 4-fluorophenylsulfonyl chloride (29.1 mg, 0.15 mmol). The HCI salt ether (32.6 mg, 74%) was prepared from the crude product using 1 M HCI in ether. m.p 256-7 °C, HRMS-FAB⁺ for C₂₀H₁₈N₂O₂SCIF·HCl, calculated MH⁺(-HCl): 405.08398; found: 405.0797.
(hh) 1 -(4-Fluorophenylsulfonyl)-3-(1-methyl-1,2,3,6-tetrahydro-4-pyridinyl)-5-trifluoromethoxyindole: from 3-(1-methyl-1,2,3,6-tetrahydro-4-pyridinyl)-5-trifluoromethoxy-1H-indole (Example 1h, 20 mg, 0.068 mmol) and 4-fluorophenylsulfonyl chloride (20.8 mg, 0.11 mmol). The HCl salt (24.0 mg, 69%) was prepared from the crude product using 1 M HCl in ether. HRMS-FAB⁺ for C₂₁H₁₈N₂O₃SF₄·HCl, calculated MH⁺(-HCl): 455.10526; found: 455.10735.
(ii) 1-(4-t-Butylphenylsulfonyl)-3-(1-methyl-1,2,3,6-tetrahydro-4-pyridinyl)-5-trifluoromethoxyindole: from 3-(1-methyl-1,2,3,6-tetrahydro-4-pyridinyl)-5-trifluoromethoxy-1 H-indole (Example 1h, 20 mg, 0.068 mmol) and 4-t-butylphenylsulfonyl chloride (24.9 mg, 0.11 mmol). The HCl salt (9.4 mg, 25%) was prepared from the crude product using 1 M HCl in ether. HRMS-FAB⁺ for C₂₅H₂₇N₂O₃SF₃HCl, calculated MH⁺(-HCl): 493.17728; found: 493.17565.
(jj) 1-(4-Chlorophenylsulfonyl)-3-(1-methyl-1,2,3,6-tetrahydro-4-pyridinyl)-5-trifluoromethoxyindole: from 3-(1-methyl-1,2,3,6-tetrahydro-4-pyridinyl)-5-trifluoromethoxy-1 H-indole (Example 1h, 20 mg, 0.068 mmol) and 4-chlorophenylsulfonyl chloride (22.5 mg, 0.11 mmol). The HCl salt (21.8 mg, 60%) was prepared from the crude product using 1M HCl in ether. HRMS-FAB⁺ for C₂₁H₁₈N₂O₃SCIF₃·HCl, calculated MH⁺(-HCl): 473.07275; found: 473.07154.
(kk) 1-(4-Methylphenylsulfonyl)-3-(1-methyl-1,2,3,6-tetrahydro-4-pyridinyl)-5-trifluoromethoxyindole: from 3-(1-methyl-1,2,3,6-tetrahydro-4-pyridinyl)-5-trifluoromethoxy-1 H-indole (Example 1h, 20 mg. 0.068 mmol) and 4-methylphenylsulfonyl chloride (20.4 mg, 0.11 mmol). The HCl salt (17.0 mg, 49%) was prepared from the crude product using 1 M HCl in ether. HRMS-FAB⁺ for C₂₂H₂₁N₂O₃SF₃·HCl, calculated MH⁺(-HCl): 451.13031; found: 451.12820.
(ll) (4-Methoxyphenylsulfonyl)-3-(1-methyl-1,2,3,6-tetrahydro-4-pyridinyl)-5-trifluoromethoxyindole: from 3-(1-methyl-1,2,3,6-tetrahydro-4-pyridinyl)-5-trifluoromethoxy-1H-indole (Example 1h, 20 mg, 0.068 mmol) and 4-methoxyphenylsulfonyl chloride (22.1 mg, 0.11 mmol). The HCI salt (15.8 mg, 44%) was prepared from the crude product using 1M HCI in ether. HRMS-FAB⁺ for C₂₂H₂₁N₂O₄SF₃·HCl, calculated MH⁺(-HCl): 467.12524; found: 467.12526.
(mm) 1-(4-Fluorophenylsulfonyl)-3-(1-methyl-1,2,3,6-tetrahydro-4-pyridinyl)-5-trifluoromethylindole hydrochloride: (15.0 mg, 44%); from 3-(1-methyl-1,2,3,6-tetrahydro-4-pyridinyl)-5-trifluoromethyl-1H-indole (Example 1i, 20 mg, 0.071 mmol) and 4-fluorophenylsulfonyl chloride (20.8 mg, 0.107 mmol). HRMS-FAB⁺ for C₂₁H₁₈N₂O₂SF₄HCl, calculated MH⁺(-HCl): 439.11035; found: 439.10807.
(nn) 1-(4-t-Butylphenylsulfonyl)-3-(1-methyl-1,2,3,6-tetrahydro-4-pyridinyl)-5-trifluoromethylindole hydrochloride: (8.7 mg, 24%); from 3-(1-methyl-1,2,3,6-tetrahydro-4-pyridinyl)-5-trifluoromethyl-1H-indole (Example 1i, 20 mg, 0.071 mmol) and 4-t-butylphenylsulfonyl chloride (24.9 mg, 0.11 mmol). HRMS-FAB⁺ for C₂₅H₂₇N₂O₂F₃·HCl, calculated MH⁺(-HCl): 477.18237; found: 477.18134.
(oo) 1-(4-Chlorophenylsulfonyl)-3-(1-methyl-1,2,3,6-tetrahydro-4-pyridinyl)-5-trifluoromethylindole hydrochloride: (11.5 mg, 33%); from 3-(1-methyl-1,2,3,6-tetrahydro-4-pyndinyl)-5-trifluoromethyl-1H-indole (Example, 1i, mg, 0.071 mmol) and 4-chlorophenylsulfonyl chloride (22.5 mg, 0.11 mmol). HRMS-FAB⁺ for C₂₁H₁₈ClF₃N₂O₂S·HCl, calculated MH⁺(-HCl): 455.08078; found: 455.08266.
(pp) 1-(4-Methoxyphenylsulfonyl)-3-(1-methyl-1,2,3,6-tetrahydro-4-pyridinyl)-5-trifluoromethylindole hydrochloride: (22.2 mg, 69%); from 3-(1-methyl-1,2,3,6-tetrahydro-4-pyridinyl)-5-trifluoromethyl-1H-indole (Example 1i, 20 mg, 0.071 mmol) and 4-methoxyphenylsulfonyl chloride (22.1 mg, 0.107 mmol). HRMS-FAB⁺ for C₂₂H₂₁N₂O₃SF₃HCl, calculated MH⁺: 451.13031; found: 451.13007.
(qq) 1-(4-Methylphenylsulfonyl)-3-(1-methyl-1,2,3,6-tetrahydro-4-pyridinyl)-5-trifluoromethylindole hydrochloride: (14.8 mg, 48%); from 3-(1-methyl-1,2,3,6-tetrahydro-4-pyridinyl)-5-trifluoromethyl-1H-indole (Example 1i, 20 mg, 0.071 mmol) and 4-methylphenylsulfonyl chloride (20.4 mg, 0.107 mmol). HRMS-FAB⁺ for C₂₂H₂₁N₂O₂SF₃HCl, calculated MH⁺ 435.13541; found 435.13700.
(rr) 5-Cyclohexyloxy-1-(4-methylphenylsulfonyl)-3-(1-methyl-4-piperidinyl)indole (12.0 mg, 51%), from 5-cyclohexyloxy-3-(1-methyl-4-piperidinyl)-1H-indole (Example 2a, 15.7 mg, 0.05 mmol) and 4-methylphenylsulfonyl chloride (50 mg, 0.26 mmol); HRMS-FAB⁺ for C₂₇H₃₄N₂O₃S: calculated MH⁺: 467.23685; found: 467.23882.
(ss) 5-Chloro-1-(4-fluorophenylsulfonyl)-3-(1-methyl-4-piperidinyl)indole (18.4 mg, 45%) from 5-chloro-3-(1-methyl-4-piperidinyl)-1H-indole (Example 2e, 25 mg, 0.1 mmol) and 4-fluorophenylsulfonyl chloride (29.2 mg, 0.15 mmol), HRMS-FAB* for C₂₀H₂₀N₂₀O₂SCIF, calculated MH⁺: 407.09964; found: 407.09685.
(tt) 1-(4-Fluorophenylsulfonyl)-3-(1-methyl-4-piperidinyl)indole: (17.0 mg, 46%), from 3-(1-methyl-4-piperidinyl)1H-indole (Example 2d, 21.5 mg, 0.1 mmol) and 4-fluorophenylsulfonyl chloride (29.2 mg, 0.15 mmol), HRMS-FAB⁺ for C₂₀H₂₁N₂O₂SF, calculated MH⁺: 373.13861; found: 373.13669.
(uu) 1-(4-Fluorophenylsulfonyl)-6-chloro-3-(1-methyl-4-piperidinyl)indole: (26.8 mg, 65%), from 6-chloro-3-(1-methyl-4-pipendinyl)-1H-indole (Example 2b, 25 mg, 0.10 mmol) and 4-fluorophenylsulfonyl chloride (29.2 mg, 0.15 mmol), HRMS-FAB⁺ for C₂₀H₂₀N₂O₂SCIF, calculated MH⁺: 407.09964; found: 407.09929.
(vv) 1-(4-Fluorophenylsulfonyl)-5-fluoro-3-(1 -methyl-4-pipendinyl)indole: (22.6 mg, 58%) from 5-fluoro-3-(1-methyl-4-piperidinyl)-1H-indole (Example 2c, 25 mg, 0.1 mmol) and 4-fluorophenylsulfonyl chloride (29.2 mg, 0.15 mmol), HRMS- FAB⁺ for C₂₀H₂₀N₂O₂SF₂, calculated MH⁺: 391.12918; found: 391.12926.
(ww) 5,7-Difluoro-3-(1-methyl-1,2,3,6-tetrahydro-4-pyridinyl)-1-(4-methylphenylsulfonyl)indole: (8.8 mg, 54%); from 5,7-difluoro-3-(1-methyl-1,2,3,6-tetrahydro-4-pyridinyl)-1H-indole (Example 1g, 10 mg, 0.04 mmol) and 4-methylphenylsulfonyl chloride (11.4 mg, 0.06 mmoles) with 1 M NaN(TMS)₂ (60*µ*L, 0.06 mmol) in THF (0.5 mL) at RT.
(xx) 5,7-Difluoro-1-(4-fluorophenylsulfonyl)-3-(1-methyl-1,2,3,6-tetrahydro-4-pyridinyl)indole: (7.6 mg, 46.4%); from 5,7-difluoro-3-(1-methyl-1,2,3,6-tetrahydro-4-pyridinyl)-1H-indole (Example 1g, 10 mg, 0.04 mmoles) and 4-fluorophenylsulfonyl chloride (11.7 mg, 0.06 mmol) with 1 M NaN(TMS)₂ (60 µL, 0.06 mmoles) in THF (0.5 mL) at RT.
(yy) 1-(4-Bromophenylsulfonyl)-5,7-difluoro-3(1-methyl-1,2,3,6-tetrahydro-4-methyl-1-pyridinyl)indole: (8.7 mg, 48%); from 5,7-difluoro-3-(1-methyl-1,2,3,6-tetrahydro-4-pyridinyl)-1H-indole (Example 1g, 10 mg, 0.04 mmol) and 4-bromophenylsulfonyl chloride (15.3 mg, 0.06 mmol) with 1 M NaN(TMS)₂ (60 µL, 0.06 mmol) in THF (0.5 mL) at RT.
(zz) 1-(4-Chlorophenylsulfonyl)-5,7-difluoro-3-(1-methyl-1,2,3,6-tetrahydro-4-pyridinyl)indole: (6.7mg, 39%); from 5,7-difluoro-3-(1-methyl-1,2,3,6-tetrahydro-4-pyridinyl)-1H-indole (Example 1g, 10 mg, 0.04mmol) and 4-chlorophenylsulfonyl chloride (12.7 mg, 0.06 mmoles) with 1M NaN (TMS)₂ (60 µL, 0.06 mmol) in THF (0.5 mL) at RT.
(aaa) 5,7-Difluoro-3-(1-methyl-1,2,3,6-tetrahydro-4-pyridinyl)-1-(1-naphthylsulfonyl)indole: (9.2 mg, 52%); from 5,7-difluoro-3-(1-methyl-1,2,3,6-tetrahydro-4-pyridinyl)-1H-indole (Example 1g, 10 mg, 0.04 mmol) and 1-naphthalenesulfonyl chloride (13.6 mg, 0.06 mmol) with 1 M NaN(TMS)₂ (60 µL, 0.06 mmol) in THF (0.5 mL) at RT.
(bbb) 5,7-Difluoro-3-(1-methyl-1,2,3,6-tetrahydro-4-pyridinyl)-(2-naphthylsulfonyl)indole: (5.1 mg, 29%); from 5,7-difluoro-3-(1-methyl-1,2,3,6-tetrahydro-4-pyridinyl)-1 H-indole (Example 1g, 10 mg, 0.04 mmol) and 2-naphthalenesulfonyl chloride (13.6 mg, 0.06 mmol) with 1 M NaN(TMS)₂ (60 *µ*L, 0.06 mmol) in THF (0.5 mL) at RT.
(ccc) 5,7-Difluoro-3-(1-methyl-1,2,3,6-tetrahydro-4-pyridinyl)-1-(2,4,6-trimethylphenyl)sulfonylindole: (5.7 mg, 33%); from 5,7-difluoro-3-(1-methyl-1,2,3,6-tetrahydro-4-pyridinyl)-1H-indole (Example 1g, 10 mg, 0.04 mmol) and 2,4,6-trimethylphenylsulfonyl chloride (13.1 mg, 0.06 mmol) with 1M NaN(TMS)₂ (60 *µ*L, 0.06 mmol) in THF (0.5 mL).

### Example 5: Binding Affinity for the 5-HT₆ Receptor

All of the compounds of the invention were evaluated using cell types receptive specifically to the 5-HT₆ receptor (for cloning and characterization of the human 5-HT₆ receptor see Kohen, et al. J. Neurochemistry, 66, 1996: 47-56). The assay protocol generally entailed the incubation of membranes prepared from cells expressing the 5-HT₆ receptor with ³H-LSD. Increasing levels of the test compound were incubated with the radioligand and the membrane homogenates prepared from the recombinant cells. After a 60 minute incubation at 37 °C, the incubation was terminated by vacuum filtration. The filters were washed with buffer and the filters were counted for radioactivity using liquid scintillation spectrometry. The affinity of the test compound for the 5-HT₆ receptor was determined by computer-assisted analysis of the data and determining the amount of the compound necessary to inhibit 50% of the binding of the radioligand to the receptor. Concentrations ranging from 10⁻¹¹ M to 10⁻⁵ M of the test compound were evaluated. For comparison, the affinity of clozapine for the 5-HT₆ receptor (Ki ~ 3 nm) was used as a standard. All of the compounds of the invention exhibited affinity for the human 5-HT₆ receptor, with Ki's of not greater than 1000 nM. Preferred compounds, those of 3b, 4a, 4b, 4c, 4d, 4e, 4f, 4g, 4i, 4k, 4z, 4aa, 4bb, 4gg, 4jj, 4kk, 4qq, 4ww, 4yy, 4zz, 4aaa, 4bbb, exhibited Ki's of not greater than 50 nM. Particularly preferred compounds, those of examples 3b, 4a, 4c, 4z, 4aa, 4bb, 4ee, 4gg, 4jj, 4kk, 4ww, 4yy, 4zz, 4aaa, 4bbb, exhibited Ki's of not greater than 5 nM. The compounds of the invention also bound to the human 5-HT₆ receptor in a selective manner, relative to the human 5-HT_{2C} and 5-HT₇ receptors. That is, the compounds of the invention bound to the human 5-HT₆ receptor with at least a 2-fold greater affinity, relative to the human 5-HT_{2C} and 5-HT₇ receptors. Preferred compounds, for example, those of examples 3b, 4i, 4z, 4bb, 4ee, 4ff, bound to the human 5-HT₆ receptor with at least a 60-fold greater affinity, relative to the human 5-HT_{2C} and 5-HT₇ receptors. More preferred compounds, for example, those of example 4ee bound to the human 5-HT₆ receptor with at least a 300-fold greater affinity, relative to the human 5-HT_{2C} and 5-HT₇ receptors.

### Example 6. Effect of Compounds on the cAMP Response of Human 5-HT₆ Receptors

The antagonist (or agonist) property of compounds for human 5-HT₆ receptors was determined by testing their effect on cAMP accumulation in stably transfected HEK293 cells.

Binding of an agonist to the human 5-HT₆ receptor will lead to a increase in adenyl cyclase activity. A compound which is an agonist will show an increase in cAMP production and a compound which is an antagonist will block the agonist effect.

*Cell Assay*: Human 5-HT₆ receptors were cloned and stably expressed in HEK293 cells. These cells were plated in 6 well plates in DMEM/F12 media with 10% fetal calf serum (FCS) and 500 µg/mL G418 and incubated at 37°C in a CO₂ incubator. The cells were allowed to grow to about 70% confluence before initiation of the experiment.

On the day of the experiment, the culture media was removed, and the cells were washed once with serum free medium (SFM). Two mL of SFM+IBMX media was added and incubated at 37°C for 10 min. The media were removed and fresh SFM+IBMX media containing various compounds, and 1 µM serotonin (as antagonist) were added to the appropriate wells and incubated for 30 min. Following incubation, the media were removed and the cells were washed once with 1 mL of PBS (phosphate buffered saline). Each well was treated with 1 mL cold 95% ethanol and 5 mM EDTA (2:1) at 4°C for 1 hour. The cells were then scraped and transferred into Eppendorf tubes. The tubes were centrifuged for 5 min at 4°C, and the supernatants were stored at 4 °C until assayed.

*cAMP Measurement:* cAMP content was determined by EIA (enzyme-immunoassay) using the Amersham Biotrak cAMP EIA kit (Amersham RPN 225). The procedure used is as described for the kit. Briefly, cAMP is determined by the competition between unlabeled cAMP and a fixed quantity of peroxidase-labelled cAMP for the binding sites on anti-cAMP antibody. The antibody is immobilized onto polystyrene microtitre wells precoated with a second antibody. The reaction is started by adding 50 µL peroxidase-labeled cAMP to the sample (100 µL) preincubated with the antiserum (100 µL) for 2 hours at 4 °C. Following 1 hour incubation at 4 °C, the unbound ligand is separated by a simple washing procedure. Then an enzyme substrate, tetramethylbenzidine (TMB), is added and incubated at room temperature for 60 min. The reaction is stopped by the addition of 100 µL 1.0 M sulfuric acid and the resultant color read by a microtitre plate spectrophotometer at 450 nM within 30 minutes.

All of the compounds of the invention which were tested in the above assays were found to be antagonists. The potency of compounds of the invention as antagonists is expressed as an EC₅₀, which is the concentration causing 50% inhibition of the serotonin-stimulated cAMP response. The table below presents the EC₅₀'s of some of the compounds of the invention compared to clozapine as a reference compound.

## Claims

1. A compound according to Formula I and salts, solvates or hydrates thereof: 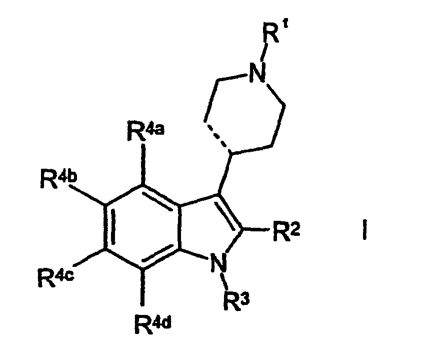 wherein:
R¹ is selected from the group consisting of H and C₁₋₄alkyl;
R² is selected from the group consisting of H, C₁₋₄alkyl and benzyl;
― represents a single or double bond;
R³ is SO₂R⁵;
R^{4a} is selected from the group consisting of H, OH, halo, C₁₋₄alkyl and C₁₋₄alkoxy;
R^{4b} is selected from the group consisting of H, hydroxy, halo,
C₃₋₇cycloalkyloxy, C₁₋₄alkoxy, C₁₋₄alkyl, benzyloxy, phenoxy, trifluoromethyl, trifluoromethoxy and vinyl;
R^{4c} is selected from the group consisting of H, OH, halo, C₁₋₄alkyl and C₁₋₄alkoxy;
R^{4d} is selected from the group consisting of H, OH, halo, C₁₋₄alkyl and C₁₋₄alkoxy; and
R⁵ is selected from the group consisting of phenyl, pyridyl, thienyl, quinolinyl and naphthyl which are optionally substituted with 1-4 substituents selected from C₁₋₄alkoxy, C₁₋₄alkyl, halo, nitro, trifluoromethyl, trifluoromethoxy, 1,2-methylenedioxy, C₁₋₄alkylcarbonyl, C₁₋₄alkoxycarbonyl and C₁₋₄alkylS.

2. A compound according to claim 1, wherein R¹ is methyl.

3. A compound according to claim 1, wherein R² is H.

4. A compound according to claim 1, wherein R⁵ is selected from the group consisting of phenyl, napthyl and thienyl which are all optionally substituted with 1-3 groups selected independently from methyl, methoxy, halo, trifluoromethyl and 1,2-methylenedioxy.

5. A compound according to claim 4, wherein R⁵ is selected from the group consisting of naphthyl and phenyl optionally substituted with 1-3 substituents independently selected from chloro, bromo, fluoro, nitro, methyl and methoxy.

6. A compound according to claim 5, wherein R⁵ is selected from the group consisting of 2-chlorophenyl, 2-bromophenyl, 1-naphthyl, 4-fluorophenyl, 4-chlorophenyl, 4-bromophenyl, 2,4-dichlorophenyl, 3-nitro-4-chlorophenyl, 2,4,6-trimethylphenyl, 4-methyoxyphenyl and 4-methylphenyl.

7. A compound according to claim 1, wherein R^{4a}, R^{4c} and R^{4d} are H and R^{4b} is selected from H, halo, methyl, trifluoromethyl and methoxy.

8. A compound according to claim 1. wherein F^{4b} and R^{4d} are halo.

9. A compound according to claim 1, wherein - represents a double bond.

10. A compound according to claim 1, which is selected from:
5-Fluoro-1-(4-methylphenylsulfonyl)-3-(1-methyl-1,2,3,6-tetrahydro-4-pyridinyl)indole;
1-(4-Methoxyphenylsulfonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridlnyl)indole;
1-(4-Bromophenylsulfonyl)-5-fluoro-3-(1-methyl-1,2,3,6-tetrahydro-4-pyridinyl)indole;
5-Fluoro-1-(2,5-dichlorophenylsulfonyl)-3-(1-methyl-12,3,6-tetrahydro-4-pyridinyl)indole;
1-(4-Chloro-3-nitrophenylsulfonyl)-5-fluoro-3-(1-methyl-1,2,3,6-tetrahydro-4-pyridinyl)indole;
5-Fluoro-3-(1-methyl-1,2,3,6-tetrahydro-4-pyridinyl)-1-(2,4,6-trimethylphenylsulfonyl)indole;
5-Fluoro-3-(1-methyl-1,2,3,6-tetrahydro-4-pyridinyl)-1-(2,4,6-triisopropylphenylsuffonyl)indole;
5-Cyclohexyloxy-1-(4-methylphenylsulfonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridinyl)-5-trifluoromethylindole hydrochloride;
1-(4-t-Butylphenylsulfonyl)-3-(1-methyl-1,2,3,6-tetrahydro-4-pyrinyl)-5-trifluoromethylindole hydrochloride;
1-(4-Chlorophenylsulfonyl)-3-(1-methyl-1,2,3,6-tetrahydro-4-pyridinyl)-5-trifluoromethylindole hydrochloride;
1-(4-Methoxyphenylsulfonyl)-3-(1-methyl-1,2,3,6-tetrahydro-4-pyridinyl)-5-trifluoromethylindole hydrochloride;
1-(4-Methylphenylsulfonyl)-3-(1-methyl-1,2,3,6-tetrahydro-4-pyridinyl)-5-trifluoromethylindole hydrochloride;
5-Cyclohexyloxy-1-(4-methylphenylsulfonyl)-3-(1-methyl-4-piperidinyl)indole;
5-Chloro-1-(4-fluorophenylsulfonyl)-3-(1-methyl-1-piperidinyl)indole;
1-(4-Fluorophenylsulfonyl)-3-(1-methyl-4-piperidinyl)indole;
1-(4-Fluorophenylsulfonyl)-6-chloro-3-(1-methyl-4-piperidinyl)indole;
1-(4-Fluorophenylsulfonyl)-5-fluoro-3-(1-methyl-4-piperidinyl)indole;
5,7-Difluoro-3-(1-methyl-1,2,3,6-tetrahydro-4-pyridinyl)-1-(4-methylphenylsulfonyl)indole;
5,7-Difluoro-1-(4-fluorophenylsulfonyl)-3-(1-methyl-1,2,3,6-tetrahydro-4-pyridinyl)indole;
1-(4-Bromophenylsulfonyl)-5,7-difluoro-3-(1-methyl-1,2,3,6-tetrahydro-4-pyridinyl)indole;
1-(4-Chlorophenylsulfonyl)-5,7-difluoro-(1-methyl-1,2,3,6-tetrahydro-4-pyridinyl)indole;
5,7-Difluoro-3-(1-methyl-1,2,3,6-tetrahydro-4-pyridinyl)-1-(1-naphthylsulfonyl)indole;
5,7-Difluoro-3-(1-methyl-1,2,3,6-tetrahydro-4-pyridinyl)-1-(2-naphthylsulfonyl)indole; and
5.7-Difluoro-3-(1-methyl-1,2,3,6-tetrahydro-4-pyridinyl)-1-(2,4,6-trimethylphenyl)sulfonylindole.

11. A compound according to claim 1, which is selected from:
5-Fluoro-1-(4-methylphenylsulfonyl)-3-(1-methyl-1,2,3,6-tetrahydro-4-pyridinyl)indole;
1-(4-Methoxyphenylsulfonyl)-3-(1-methyl-1,2,3,6-tetrahydro-4-pyridinyl)indole;
1-(4-Fluorophenylsulfonyl)-3-(1-methyl-1,2,3,6-tetrahydro-4-pyridinyl)indole;
1-(4-Brumophenylsulfonyl)-5-fluoro-(1-methyl-1,2,3,6-tetrahydro-4-pyridinyl)indole;
5-Fluoro-1-(2,5-dichlorophenylsulfonyl)-3-(1-methyl-1,2,3,6-tetrahydro-4-pyridinyl)indole;
5-Chloro-1-(4-fluorophenylsulfonyl)-3-(1-methyl-1,2,3,6-tetrahydro-4-pyridinyl)indole;
1-(4-Chlorophenylsulfonyl)-3-(1-methyl-1,2,3,6-tetrahydro-4-pyridinyl)-5-trifluoromethoxyindole;
1-(4-Methylphenylsulfonyl)3-(1-methyl-1,2,3,6-tetrahydro-4-pyridiny)-5-trifluoromethoxyindole;
1-(4-Chlorophenylsulfonyl)-3-(1-methyl-1,2,3,6-tetrahydro-4-pyridinyl)-5-trifluoromethylindole hydrochloride;
1-(4-Methoxyphenylsulfonyl)-3-(1-methyl-1,2,3,6-tetrahydro-4-pyridinyl)-5-trifluoromethylindole hydrochloride;
1-(4-Methylphenylsulfonyl)-3-(1-methyl-1,2,3,6-tetrahydro-4-pyridinyl)-5-trifluoromethylindole hydrochloride;
5,7-Difluoro-3-(1-methyl-1,2,3,6-tetrahydro-4-pyridinyl)-1-(4-methylphenylsulfonyl)indole;
1-(4-Bromophenylsulfonyl)-5,7-difluoro-3-(1-methyl-1,2,3,6-tetrahydro-4-pyridinyl)indole;
1-(4-Chlorophenylsulfonyl)-5,7-difluoro-3-(1-methyl-1,2,3,6-tetrahydro-4-pyridinyl)indole;
5,7-difluoro-3-(1-methyl-1,2,3,6-tetrahydro-4-pyridinyl)-1-(2-naphthylsulfonyl)indole; and
5,7-Difluoro-3-(1-methyl-1,2,3,6-tetrahydro-4-pyridinyl)-1-(1-naphthylsulfonyl)indole.

12. A compound according to claim 1, which is selected from:
5-Fluoro-1-(4-methylphenylsulfonyl)-3-(1-methyl-1,2,3,6-tetrahydro-4-pyridinyl)indole,
1-(4-Fluorophenylsulfonyl)-3-(1-methyl-1,2,3,8-tetrahydro-4-pyridinyl)indole;
1-(2-Bromophenylsulfonyl)-5-fluoro-3-(1-methyl-1,2,3,6-tetrahydro-4-pyridinyl)indole;
1-(4-Fluorophenylsulfonyl)-5-fluoro-3-(1-methyl-1,2,3,6-tetrahydro-4-pyridinyl)indole;
1-(4-Chlorophenylsulfonyl)-5-fluoro-3-(1-methyl-1,2,3,6-tetrahydro-4-pyridinyl)indole;
5-Fluoro-3-(1-methyl-1,2,3,6-tetrahydro-4-pyridinyl)-1-(1-naphthylsulfonyl)indole;
5-Chloro-1-(4-fluorophenylsulfonyl)-3-(1-methyl-1,2,3,6-tetrahydro-4-pyridinyl)indole;
1-(4-Chlorophenylsulfonyl)-3-(1-methyl-1,2,3,6-tetrahydro-4-pyridinyl)-5-trifluoromethoxyindole;
1-(4-Methylphenylsulfonyl)-3-(1-methyl-1,2,3,6-tetrahydro-4-pyridinyl)-5-trifluoromethoxyindole;
5,7-Difluoro-3-(1-methyl-1,3,6-tetrahydro-4-pyridinyl-1-(4-methylphenylsulfony)Indole;
1-(4-Bromophenylsulfonyl)-5,7-difluoro-3-(1-methyl-1,2,3,6-tetrahydro-4-pyridinyl)indole;
1-(4-Chlorophenylsulfonyl)-5,7-difluoro-3-(1methyl-1,2,3,6-tetrahydro-4-pyridinyl)indole;
5,7-Difluoro-3-(1-methyl)-1,2,3,6-tetrahydro-4-pyridinyl)-1-(1-naphthylsulfonyl)indole; and
5,7-Difluoro-3-(1-methyl-1,2,3,6-tetrahydro-4-pyridinyl)-1-(2-naphthylsulfonyl)indole.

13. The use of the compound of any one of Claims 1 to 12 in the manufacture of a medicament for the treatment or prevention of central nervous system disturbances.

14. The use of claim 13 wherein the central nervous system disturbances are psychosis, schizophrenia, manic depression, depression, Parkinsonism, neurological disturbances, memory disturbances, amylotropic lateral sclerosis, Alzheimer's disease or Huntington's disease.

## Patentansprüche

1. Verbindung nach Formel I und Salze, Solvate oder Hydrate davon: 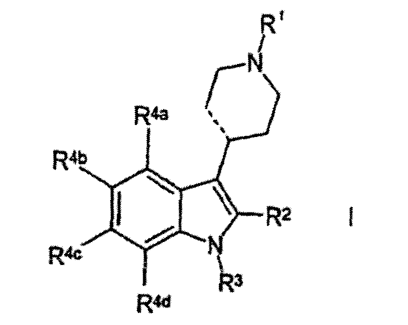 worin:
R¹ ausgewählt ist aus der Gruppe bestehend aus H und C₁₋₄-Alkyl;
R² ausgewählt ist aus der Gruppe bestehend aus H, C₁₋₄-Alkyl und Benzyl;
--- eine Einfach- oder Doppelbindung bedeutet;
R³ SO₂R⁵ bedeutet;
R^{4a} ausgewählt ist aus der Gruppe bestehend aus H, OH, Halogen, C₁₋₄-Alkyl und C₁₋₄-Alkoxy;
R^{4b} ausgewählt ist aus der Gruppe bestehend aus H, Hydroxy, Halogen, C₃₋₇-Cycloalkyloxy, C₁₋₄-Alkoxy, C₁₋₄-Alkyl, Benzyloxy, Phenoxy, Trifluormethyl, Trifluormethoxy und Vinyl;
R^{4c} ausgewählt ist aus der Gruppe bestehend aus H, OH, Halogen, C₁₋₄-Alkyl und C₁₋₄-Alkoxy;
R^{4d} ausgewählt ist aus der Gruppe bestehend aus H, OH, Halogen, C₁₋₄-Alkyl und C₁₋₄-Alkoxy; und
R⁵ ausgewählt ist aus der Gruppe bestehend aus Phenyl, Pyridyl, Thienyl, Chinolinyl und Naphthyl, welche gegebenenfalls substituiert sind mit 1-4 Substituenten, ausgewählt aus C₁₋₄-Alkoxy, C₁₋₄-Alkyl, Halogen, Nitro, Trifluormethyl, Trifluormethoxy, 1,2-Methylendiaxy, C₁₋₄-Alkylcarbonyl, C₁₋₄-Alkoxycarbonyl und C₁₋₄-AlkylS-.

2. Verbindung nach Anspruch 1, worin R¹ Methyl ist.

3. Verbindung nach Anspruch 1, worin R² H ist.

4. Verbindung nach Anspruch 1, worin R⁵ ausgewählt ist aus der Gruppe bestehend aus Phenyl, Naphthyl und Thienyl, welche alle gegebenenfalls mit 1-3 Gruppen substituiert sind, die unabhängig voneinander ausgewählt sind aus Methyl, Methoxy, Halogen, Trifluormethyl und 1,2-Methylendioxy.

5. Verbindung nach Anspruch 4, worin R⁵ ausgewählt ist aus der Gruppe bestehend aus Naphthyl und Phenyl, gegebenenfalls substituiert mit 1-3 Substituenten, die unabhängig voneinander ausgewählt sind aus Chlor, Brom, Fluor, Nitro, Methyl und Methoxy.

6. Verbindung nach Anspruch 5, worin R⁵ ausgewählt ist aus der Gruppe bestehend aus 2-Chlorphenyl, 2-Bromphenyl, 1-Naphthyl, 4-Fluorphenyl, 4-Chlorphenyl, 4-Bromphenyl, 2,4-Dichlorphenyl, 3-Nitro-4-chlorphenyl, 2,4,6- Trimethylphenyl, 4-Methyoxyphenyl und 4-Methylphenyl.

7. Verbindung nach Anspruch 1, worin R^{4a}, R^{4c} und R^{4d} H sind und R^{4b} ausgewählt ist aus H, Halogen, Methyl, Trifluormethyl und Methoxy.

8. Verbindung nach Anspruch 1, worin R^{4b} und R^{4d} Halogen sind.

9. Verbindung nach Anspruch 1, worin --- eine Doppelbindung bedeutet.

10. Verbindung nach Anspruch 1, welche ausgewählt ist aus:
5-Fluor-1-(4-methylphenylsulfonyl)-3-(1-methyl-1,2,3,6-tetrahydro-4-pyridinyl)indol;
1-(4-Methoxyphenylsulfonyl)-3-(1-methyl-1,2,3,6-tetrahydro-4-pyridinyl)indol;
1-(4-Fluorphenylsulfonyl)-3-(1-methyl-1,2,3,6-tetrahydro-4-pyridinyl)indol;
1-(4-Bromphenylsulfonyl)-5-fluor-3-(1 -methyl-1,2,3,6-tetrahydro-4-pyridinyl)indol;
5-Fluor-1-(2,5-dichlorphenylsulfonyl)-3-(1-methyl-1,2,3,6-tetrahydro-4-pyridinyl)indol;
1-(4-Chlor-3-nitrophenylsulfonyl)-5-fluor-3-(1-methyl-1,2,3,6-tetrahydro-4-pyridinyl)indol;
5-Fluor-3-(1 -methyl-1,2,3,6-tetrahydro-4-pyridinyl)-1-(2,4,6-trimethylphenylsulfonyl)indol;
5-Fluor-3-(1-methyl-1,2,3,6-tetrahydro-4-pyridinyl)-1-(2,4,6-triisopropylphenylsulfonyl)indol;
5-Cyclohexyloxy-1-(4-methylphenylsulfonyl)-3-(1-methyl-1,2,3,6-tetrahydro-4-pyridinyl)indol;
1-(4-Fluorphenylsulfonyl)-3-(1-methyl-1,2,3,6-tetrahydro-4-pyridinyl)-5-trifluormethylindol-hydrochlorid;
1-(4-t-Butylphenylsulfonyl)-3-(1-methyl-1,2, 3,6-tetrahydro-4-pyridinyl)-5-trifluormethylindol-hydrochlorid;
1-(4-Chlorphenylsulfonyl)-3-(1-methyl-1,2,3,6-tetrahydro-4-pyridinyl)-5-trifluormethylindol-hydrochlorid;
1-(4-Methoxyphenylsulfonyl)-3-(1-methyl-1,2,3,6-tetrahydro-4-pyridinyl)-5-trifluormethylindol-hydrochlorid;
1-(4-Methylphenylsulfonyl)-3-(1-methyl-1,2,3,6-tetrahydro-4-pyridinyl)-5-trifluormethylindol-hydrochlorid;
5-Cyclohexylaxy-1-(4-methylphenylsulfonyl)-3-(1-methyl-4-piperidinyl)indol;
5-Chlor-1 -(4-fluorphenylsulfonyl)-3-(1 -methyl-4-piperidinyl)indol;
1-(4-Fluorphenylsulfonyl)-3-(1-methyl-4-piperidinyl)indol;
1-(4-Fluorphenylsulfonyl)-6-chlor-3-(1-methyl-4-piperidinyl)indol;
1-(4-Fluorphenylsulfonyl)-5-fluor-3-(1-methyl-4-piperidinyl)indol;
5,7-Difluor-3-(1 -methyl-1,2,3,6-tetrahydro-4-pyridinyl)-1-(4-methylphenylsulfonyl)indol;
5,7-Difluor-1-(4-fluorphenylsulfonyl)-3-(1-methyl-1,2,3,6-tetrahydro-4-pyridinyl)indol;
1-(4-Bromphenylsulfonyl)-5,7-difluor-3-(1-methyl-1,2,3,6-tetrahydro-4-pyridinyl)indol;
1-(4-Chlorphenylsulfonyl)-5,7-difluor-3-(1 -methyl-1,2,3,6-tetrahydro-4-pyridinyl)indol;
5,7-Difluor-3-(1-methyl-1,2,3,6-tetrahydro-4-pyridinyl)-1-(1-naphthylsulfonyl)indol;
5,7-Difluor-3-(1-methyl-1,2,3,6-tetrahydro-4-pyridinyl)-1-(2-naphthylsulfonyl)indol; und
5,7-Difluor-3-(1-methy(-1,2,3,6-tetrahydro-4-pyridinyl)-1-(2,4,6-trimethylphenyl)sulfonylindol.

11. Verbindung nach Anspruch 1, welche ausgewählt ist aus:
5-Fluor-1-(4-methylphenylsulfonyl)-3-(1-methyl-1,2,3,6-tetrahydro-4-pyridinyl)indol;
1-(4-Methoxyphenylsulfonyl)-3-(1-methyl-1,2,3,6-tetrahydro-4-pyridinyl)indol;
1-(4-Fluorphenylsulfonyl)-3-(1-methyl-1,2,3,6-tetrahydro-4-pyridinyl)indol;
1-(4-Bromphenylsulfonyl)-5-fluor-3-(1-methyl-1,2,3,6-tetrahydro-4-pyridinyl)indol;
5-Fluor-1-(2,5-dichlorphenylsulfonyl)-3-(1-methyl-1,2,3,6-tetrahydro-4-pyridinyl)indol;
5-Chlor-1-(4-fluorphenylsulfonyl)-3-(1-methyl-1,2,3,6-tetrahydro-4-pyridinyl)indol;
1-(4-Chlorphenylsulfonyl)-3-(1-methyl-1,2,3,6-tetrahydro-4-pyridinyl)-5-trifiuormethoxyindol;
1-(4-Methylphenylsulfonyl)-3-(1-methyl-1,2,3,6-tetrahydro-4-pyridinyl)-5-trifluormethoxyindol;
1-(4-Chlorphenylsulfonyl)-3-(1-methyl-1,2,3,6-tetrahydro-4-pyridinyl)-5-trifluormethylindol-hydrochlorid;
1-(4-Methoxyphenylsulfonyl)-3-(1-methyl-1,2,3,6-tetrahydro-4-pyridinyl)-5-trifluormethylindol-hydrochlorid;
1-(4-Methylphenylsulfonyl)-3-(1-methyl-1,2,3,6-tetrahydro-4-pyridinyl)-5-trifluormethylindol-hydrochlorid;
5,7-Difluor-3-(1-methyl-1,2,3,6-tetrahydro-4-pyridinyl)-1-(4-methylphenylsulfonyl)indol;
1-(4-Bromphenylsulfonyl)-5,7-difluor-3-(1-methyl-1,2,3,6-tetrahydro-4-pyridinyl)indol;
1-(4-Chlorphenylsulfonyl)-5,7-difluor-3-(1-methyl-1,2, 3,6-tetrahydro-4-pyridinyl)indol;
5,7-Difluor-3-(1-methyl-1,2,3,6-tetrahydro-4-pyridinyl)-1-(2-naphthylsulfonyl)indol; und
5,7-Difluor-3-(1-methyl-1,2,3,6-tetrahydro-4-pyridinyl)-1-(1-naphthylsulfonyl)indol.

12. Verbindung nach Anspruch 1, welche ausgewählt ist aus:
5-Fluor-1-(4-methylphenylsulfonyl)-3-(1-methyl-1,2,3,6-tetrahydro-4-pyridinyl)indol;
1-(4-Fluorphenylsulfonyl)-3-(1-methyl-1,2,3,6-tetrahydro-4-pyridinyl)indol;
1-(2-Bromphenylsulfonyl)-5-fluor-3-(1-methyl-1,2,3,6-tetrahydro-4-pyridinyl)indol;
1-(4-Fluorphenylsulfonyl)-5-fluor-3-(1-methyl-1,2,3,6-tetrahydro-4-pyridinyl)indol;
1-(4-Chlorphenylsulfonyl)-5-fluor-3-(1-methyl-1,2,3,6-tetrahydro-4-pyridinyl)indol;
5-Fluor-3-(1-methyl-1,2,3,6-tetrahydro-4-pyridinyl)-1-(1-naphthylsulfonyl)indol;
5-Chlor-1-(4-fluorphenylsulfonyl)-3-(1-methyl-1,2,3,6-tetrahydro-4-pyridinyl)indol;
1-(4-Chlorphenylsulfonyl)-3-(1-methyl-1,2,3,6-tetrahydro-4-pyridinyl)-5-trifluormethoxyindol;
1-(4-Methylphenylsulfonyl)-3-(1-methyl-1,2,3,6-tetrahydro-4-pyridinyl)-5-trifluormethoxyindol;
5,7-Difluor-3-(1-methyl-1,2,3,6-tetrahydro-4-pyridinyl)-1-(4-methylphenylsulfonyl)indol;
1-(4-Bromphenylsulfonyl)-5,7-difluor-3-(1-methyl-1,2,3,6-tetrahydro-4-pyridinyl)indol;
1-(4-Chlorphenylsulfonyl)-5,7-difluor-3-(1-methyl-1,2,3,6-tetrahydro-4-pyridinyl)indol;
5,7-Difluor-3-(1-methyl-1,2,3,6-tetrahydro-4-pyridinyl)-1-(1-naphthylsulfonyl)indol; und
5,7-Difluor-3-(1-methyl-1,2,3,6-tetrahydro-4-pyridinyl)-1-(2-naphthylsulfonyl)indol.

13. Verwendung der Verbindung nach einem der Ansprüche 1 bis 12 bei der Herstellung eines Medikaments für die Behandlung oder Verhütung von Störungen des Zentralnervensystems.

14. Verwendung nach Anspruch 13, wobei die Störungen des Zentralnervensystems eine Psychose, Schizophrenie, manische Depression, Depression, die Parkinson-Krankheit, neurologische Störungen, Gedächtnisstörungen, die amyotrophe Lateralsklerose, die Alzheimer-Krankheit oder Chorea Huntington sind.

## Revendications

1. Composé selon la formule (I) et ses sels, solvates ou hydrates : 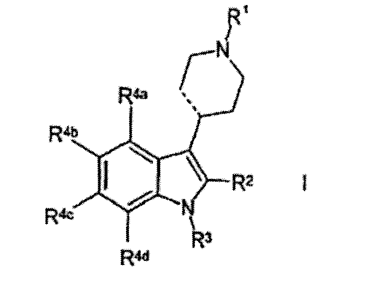 dans laquelle :
R¹ est choisi dans le groupe constitué de H et C₁₋₄ alkyle ;
R² est choisi dans le groupe constitué de H, C₁₋₄ alkyle et benzyle ;
---- représente une liaison simple ou double ;
R³ est SO₂R⁵;
R^{4a} est choisi dans le groupe constitué de H, OH, halo, C₁₋₄ alkyle et C₁₋₄ alcoxy ;
R^{4b} est choisi dans le groupe constitué de H, hydroxy, halo, C₃₋₇ cycloalkyloxy, C₁₋₄ alcoxy, C₁₋₄ alkyle, benzyloxy, phénoxy, trifluorométhyle, trifluorométhoxy et vinyle ;
R^{4c} est choisi dans le groupe constitué de H, OH, halo, C₁₋₄ alkyle et C₁₋₄ alcoxy ;
R^{4d} est choisi dans le groupe constitué de H, OH, halo, C₁₋₄ alkyle et C₁₋₄ alcoxy; et
R₅ est choisi dans le groupe constitué de phényle, pyridyle, thiényle, quinolinyle et naphtyle qui sont éventuellement substitués par 1-4 substituants choisis parmi C₁₋₄ alcoxy, C₁₋₄ alkyle, halo, nitro, trifluorométhyle, trifluorométhoxy, 1,2-méthylènedioxy, C₁₋₄ alkylcarbonyle, C₁₋₄ alcoxycarbonyle et C₁₋₄ alkyleS.

2. Composé selon la revendication 1, dans lequel R¹ est méthyle.

3. Composé selon la revendication 1, dans lequel R² est H.

4. Composé selon la revendication 1, dans lequel R⁵ est choisi dans le groupe constitué de phényle, naphtyle et thiényle qui sont tous éventuellement substitués par 1-3 groupes choisis de manière indépendante parmi méthyle, méthoxy, halo, tritluorométhyle et 1,2-méthylènedioxy.

5. Composé selon la revendication 4, dans lequel R⁵ est choisi dans le groupe constitué de naphtyle et phényle éventuellement substitués par 1-3 substituants choisis de manière indépendante parmi le chlore, brome, fluor, nitro, méthyle et méthoxy.

6. Composé selon la revendication 5, dans lequel R⁵ est choisi dans le groupe constitué de 2-chlorophényle, 2-bromophényle, 1-naphtyle, 4-fluorophényle, 4-chlorophényle, 4-bromophényle, 2,4-dichlorophényle, 3-nitro-4-chlorophényle, 2,4,6-triméthylphényle, 4-méthoxyphényle et 4-méthylphényle.

7. Composé selon la revendication 1, dans lequel R^{4a}, R^{4c} et R^{4d} sont H et R^{4b} est choisi parmi H, halo, méthyle, trifluorométhyle et méthoxy.

8. Composé selon la revendication 1, dans lequel R^{4b} et R^{4d} sont halo.

9. Composé selon la revendication 1, dans lequel ---- représente une double liaison.

10. Composé selon la revendication 1, qui est choisi parmi :
5-Fluoro-1-(4-méthylphénylsulfonyl)-3-(1-méthyl-1,2,3,6-tétrahydro-4-pyridinyl)indole;
1-(4-Méthoxyphénylsulfonyl)-3-(1-méthyl-1,2,3,6-tétrahydro-4-pyridinyl)indole;
1-(4-Fluorophénylsulfonyl)-3-(1-méthyl-1,2,3,6-tétrahydro-4-pyridinyl)indole;
1-(4-Bromophénylsulfonyl)-5-fluoro-3-(1-méthyl-1,2,3,6-tétrahydro-4-pyridinyl)indole;
5-Fluoro-1-(2,5-dichlorophénylsulfonyl)-3-(1-méthyl-1,2,3,6-tétrahydro-4-pyridinyl)indole;
1-(4-Chloro-3-nitrophénylsulfonyl)-5-fluoro-3-(1-méthyl-1,2,3,6-tétrahydro-4-pyridinyl)indole;
5-Fluoro-3-(1-méthyl-1,2,3,6-tétrahydro-4-pyridinyl)-1-(2,4,6-triméthylphénylsulfonyl)indole;
5-Fluoro-3-(1-méthyl- 1,2,3,6-tétrahydro-4-pyridinyl)-1-(2,4,6-triisopropylphénylsulfonyl)indole;
5-Cyclohexyloxy- 1 -(4-méthylphénylsulfonyl)-3-(1-méthyl-1,2,3,6-tétrahydro-4-pyridinyl)indole;
Chlorhydrate de 1-(4-Fluorophénylsulfonyl)-3-(1-méthyl-1,2,3,6-tétrahydro-4-pyridinyl)-5-trifluorométhylindole;
Chlorhydrate de 1-(4-t-Butylphénylsulfonyl)-3-(1-méthyl-1,2,3,6-tétrahydro-4-pyridinyl)-5-trifluorométhylindole;
Chlorhydrate de 1-(4-Chlorophénylsulfonyl)-3-(1-méthyl-1,2,3,6-tétrahydro-4-pyridinyl)-5-trifluorométhylindole;
Chlorhydrate de 1-(4-Méthoxyphénylsulfonyl)-3-(1-méthyl-1,2,3,6-tétrahydro-4-pyridinyl)-5-trifluorométhylindole;
Chlorhydrate de 1-(4-Méthylphénylsulfonyl)-3-(1-méthyl-1,2,3,6-tétrahydro-4-pyridinyl)-5-trifluorométhylindole;
5-Cyclohexyloxy-1-(4-méthylphénylsulfonyl)-3-(1-méthyl-4-piperidinyl)indole;
5 -Chloro-1-(4-fluorophénylsulfonyl)-3-(1-méthyl-4-piperidinyl)indole;
1-(4-Fluorophénylsulfonl)-3-(1-méthyl-4-piperidinyl)indole ;
1-(4-Fluorophénylsulfonyl)-6-chloro-3-(1-méthyl-4-piperidinyl)indole;
1-(4-Fluorophénylsulfonyl)-5-fluoro-3-(1-méthyl-4-piperidinyl)indole;
5,7-Difluoro-3-(1-méthyl-1,2,3,6-tétrahydro-4-pyridinyl)-1-(4-méthylphénylsulfonyl)indole;
5,7-Difluoro-1-(4-fluorophénylsulfonyl)-3-(1-méthyl-1,2,3,6-tétrahydro-4-pyridinyl)indole;
1-(4-Bromophénylsulfonyl)-5,7-difluoro-3-(1-méthyl-1,2,3,6-tétrahydro-4-pyridinyl)indole;
1-(4-Chlorophénylsulfonyl)-5,7-difluoro-3-(1-méthyl-1,2,3,6-tétrahydro-4-pyridinyl)indole;
5,7-Difluoro-3-(1-méthyl-1,2,3,6-tétrahydro-4-pyridinyl)-1-(1-naphthylsulfonyl)indole;
5,7-Difluoro-3-(1-méthyl-1,2,3,6-tétrahydro-4-pyridinyl)-1-(2-naphthylsulfonyl)indole; et
5,7-Difluoro-3-(1-méthyl-1,2,3,6-tétrahydro-4-pyridinyl)-1-(2,4,6-triméthylphényl)sulfonylindole.

11. Composé selon la revendication 1, qui est choisi parmi :
5-Fluoro-1-(4-méthylphénylsulfonyl)-3-(1-méthyl-1,2,3,6-tétrahydro-4-pyridinyl)indole ;
1-(4-Méthoxyphénylsulfonyl)-3-(1-méthyl-1,2,3,6-tétrahydro-4-pyridinyl) indole;
1-(4-Fluorophénylsulfonyl)-3-(1-méthyl-1 ,2,3,6-tétrahydro-4-pyridinyl)indole;
1-(4-Bromophénylsulfonyl)-5-fluoro-3-(1-méthyl-1,2,3,6-tétrahydro-4-pyridinyl)indole ;
5-Fluoro-1-(2,5-dichlorophénylsulfonyl)-3-(1-méthyl-1,2,3,6-tétrahydro-4-pyridinyl)indole;
5-Chloro-1-(4-fluorophénylsulfonyl)-3-(1-méthyl-1,2,3,6-tétrahydro-4-pyridinyl)indole;
1-(4-Chlorophénylsulfonyl)-3-(1-méthyl-1,2,3,6-tétrahydro-4-pyridinyl)-5-trifluorométhoxyindole;
1-(4-Méthylphénylsulfonyl)-3-(1-méthyl-1,2,3,6-tétrahydro-4-pyridinyl)-5-trifluorométhoxyindole;
Chlorhydrate de 1-(4-chlorophénylsulfonyl)-3-(1-méthyl-1,2,3,6-tétrahydro-4-pyridinyl)-5-trifluorométhylindole;
Chlorhydrate de 1-(4-méthoxyphenylsulfonyl)-3-(1-méthyl-1,2,3,6-tétrahydro-4-pyridinyl)-5-trifluorométhylindole;
Chlorhydrate de 1-(4-méthylphénylsulfony)-3-(1-méthyl-1,2,3,6-tétrahydro-4-pyridinyl)-5-trifluorométhylindole;
5,7-Difluoro-3-(1-méthyl-1,2,3,6-tétrahydro-4-pyridinyl)-1-(4-methylphénylsulfonyl)indole;
1-(4-Bromophénylsuflonyl)-5,7-difluoro-3-(1-méthyl-1,2,3,6-tétrahydro-4-pyridinyl)indole;
1-(4-Chlorophénylsulfonyl)-5,7-difluoro-3-(1-methyl-1,2,3,6-tétrahydro-4-pyridinyl)indole;
5,7-Difluoro-3-(1-méthyl-1,2,3,6-tétrahydro-4-pyridinyl)-1-(2-naphthylsulfonyl)indole; et
5,7-Difluoro-3-(1-méthyl-1,2,3,6-tétrahydro-4-pyridinyl)-1-(1-naphtylsulfonyl)indole.

12. Composé selon la revendication 1, qui est choisi parmi:
5 -Fluoro-1-(4-méthylphénylsulfonyl)-3-(1-méthyl-1,2,3,6-tétrahydro-4-pyridinyl)indole ;
1-(4-Fluorophénylsulfonyl)-3-(1-méthyl-1 ,2,3,6-tétrahydro-4-pyridinyl)indole;
1-(2-Bromophénylsulfonyl)-5-fluoro-3-(1-méthyl-1,2,3,6-tétrahydro-pyridinyl)indole;
1-(4-Fluorophénylsulfonyl)-5-fluoro-3-(1-méthyl-1,2,3,6-tétrahydro-4-pyridinyl)indole;
1-(4-Chlorophénylsulfonyl)-5-fluoro-3-(1-méthyl-1,2,3,6-tétrahydro-4-pyridinyl)indole;
5-Fluoro-3-(1-méthyl-1,2,3,6-tétrahydro-4-pyridinyl)-1-(1-naphthylsulfonyl)indole;
5-Chloro-1-(4-fluorophénylsulfonyl)-3-(1-méthyl-1,2,3,6-tétrahydro-4-pyridinyl)indole;
1-(4-Chlorophénylsulfonyl)-3-(1-méthyl-1,2,3,6-tétrahydro-4-pyridinyl)-5-trifluorométhoxyindole;
1-(4-Méthylphénylsulfonyl)-3-(1-méthyl-1,2,3,6-tétrahydro-4-pyridinyl)-5-trifluorométhoxyindole;
5,7-Difluoro-3-(1-méthyl-1,2,3,6-tétrahydro-4-pyridinyl)-1-(4-méthylphénylsulfonyl)indole;
1-(4-Bromophénylsulfonyl)-5,7-difluoro-3-(1-méthyl-1,2,3,6-tétrahydro-4-pyridinyl)indole;
1-(4-Chlorophénylsulfonyl)-5,7-difluoro-3-(1-méthyl-1,2,3-6-tétrahydro-4-pyridinyl) indole;
5,7-Difluoro-3-(1-méthyl-1,2,3,6-tétrahydro-4-pyridinyl)-1-(1-naphthylsulfonyl)indole; et
5,7-Difluoro-3-(1-méthyl-1,2,3,6-tétrahydro-4-pyridin)-1-(2-naphthylsulfonyl)indole.

13. Utilisation d'un composé selon l'une quelconque des revendications 1 à 12, dans la fabrication d'un médicament pour le traitement ou la prévention de troubles du système nerveux central.

14. Utilisation selon la revendication 13, dans laquelle les troubles du système nerveux central sont la psychose, la schizophrénie, la dépression nerveuse, la dépression, le syndrome parkinsonien, les troubles neurologiques, les troubles de la mémoire, la sclérose latérale amyotrophique, la maladie d'Alzheimer ou la chorée de Huntington.
